(19) Europäisches Patentamt
European Patent Office
Office européen des brevets

(11) Publication number: **0 421 436 A2**

# (12) EUROPEAN PATENT APPLICATION

(21) Application number: **90119077.7**

(51) Int. Cl.5: **C07F 9/62**, A61K 31/675

(22) Date of filing: **04.10.90**

(30) Priority: **03.10.89 US 416684**

(43) Date of publication of application:
**10.04.91 Bulletin 91/15**

(84) Designated Contracting States:
**AT BE CH DE DK ES FR GB GR IT LI LU NL SE**

(71) Applicant: **WARNER-LAMBERT COMPANY**
**201 Tabor Road**
**Morris Plains New Jersey 07950(US)**

(72) Inventor: **Johnson, Graham**
**1130 Bandera Drive**
**Ann Arbor, Michigan 48103(US)**
Inventor: **Malone, Thomas Charles**
**45139 North Spring Drive**
**Canton, Michigan 48187(US)**

(74) Representative: **Mansmann, Ivo**
**c/o Gödecke AG - Patentabteilung Postfach**
**569 Mooswaldallee 1-9**
**W-7800 Freiburg(DE)**

## (54) Substituted carboxytetrahydroisoquinolines and derivatives thereof having pharmaceutical activity.

(57) The invention includes a novel series of substituted aryl or heteroaryl fused 2- or 6-carboxy piperidines and derivatives thereof which are useful in the treatment of cerebrovascular disorders, epilepsy, Huntington's disease, or as anesthetics particularly in surgical processes where a finite risk of cerebrovascular damage exists. Processes for making the compounds, novel intermediates useful in the processes, methods for using, and compositions containing the compounds are also included.

EP 0 421 436 A2

# SUBSTITUTED CARBOXYTETRAHYDROISOQUINOLINES AND DERIVATIVES THEREOF HAVING PHARMACEUTICAL ACTIVITY

## BACKGROUND OF THE INVENTION

A series of aliphatic amino acids are disclosed in British Patent 2,104,078. This includes 2-amino-7-phosphonoheptanoic acid (APH) which is useful for the treatment of diseases of the central nervous system. British Patent 2,156,818 discloses the use of somewhat similar compounds for the treatment of epilepsy, disorders associated with GH or LH secretion, schizophrenia, depression, CNS degenerative disorders, and cerebral hypoxic conditions.

A series of unsaturated amino acids which are antagonists of excitatory amino acid receptors sensitive to NMDA is disclosed in European Patent Application 0233154.

A related series of compounds having activity as anticonvulsants, analgesics, and cognition enhancers through the antagonism of specific excitatory amino acid neurotransmitter receptors is disclosed in United States Patent 4,657,899.

A series of compounds useful for treating epilepsy and including anticonvulsant activity shown from inhibition of NMDA in excitatory amino acid systems is disclosed in British Patent Application 2,198,134.

A series of heteroalkyl phosphonic acid derivatives of 2-piperidine or 2-tetrahydropyridine carboxylates and esters thereof which are useful for the treatment of disorders responsive to blockade of NMDA receptors in mammals is disclosed in United States Patent 4,746,653.

None of the above discloses the named series of compounds covered by the instant invention.

## SUMMARY OF THE INVENTION

The present invention relates to novel 1-carboxy or 3-carboxy substituted 1,2,3,4-tetrahydroisoquinolines and derivatives thereof useful as pharmaceutical agents, to methods for their preparation, to pharmaceutical compositions which include these compounds and a pharmaceutically acceptable carrier, and to the use of these compounds for the preparation of pharmaceutical compositions. More particularly, the novel compounds of the present invention selectively block the N-methyl-D-aspartate (NMDA) excitatory amino acid receptors in mammals. Thus, the compounds of the present invention are useful for treating diseases responsive to blockade of excitatory amino acid receptors. Thus, the compounds of the present invention are useful in the treatment of cerebrovascular disorders such as cerebral ischemia, or cerebral infarction resulting from a range of conditions such as thromboembolic or hemorrhagic stroke, cerebral vasospasm, hypoglycemia, cardiac arrest, status epilepticus, and cerebral trauma. Additionally, the compounds of the present invention are useful in the treatment of schizophrenia, epilepsy, neurodegenerative disorders, Alzheimer's disease, or Huntington's disease. Further, the compounds of the present invention are useful as anesthetics, particularly in surgical procedures where a finite risk of cerebrovascular damage exists.

The present invention includes compounds of formula

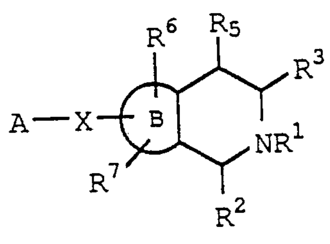

I

or a pharmaceutically acceptable acid addition or base salt thereof wherein $R^1$, $R^2$, $R^3$, $R^5$, $R^6$, $R^7$, X, B, and A are as described hereinbelow.

The present invention also includes a pharmaceutical composition comprising a therapeutically effective amount of a compound of formula I together with a pharmaceutically acceptable carrier.

The present invention also includes the use of a compound of formula I for the preparation of

pharmaceuticals for treating cerebrovascular disorders, disorders responsive to the blockade of glutamic or aspartic acid receptors, cerebral ischemia, cerebral infarction, cerebral vasospasm, hypoglycemia, cardiac arrest, status epilepticus, cerebral trauma, schizophrenia, epilepsy, neurodegenerative disorders, Alzheimer's disease, Huntington's disease or for using as an anesthetic or in surgical operations where risk of cerebrovascular damage exists.

The invention further includes processes for the preparation of compounds of formula I.

The invention still further includes novel intermediates useful in the processes.

## DETAILED DESCRIPTION

The present invention provides compounds of formula

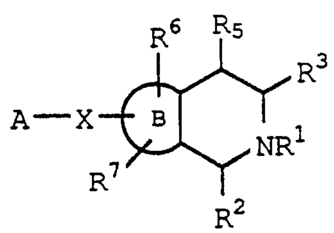

I

or a pharmaceutically acceptable acid addition or base salt thereof wherein

$R^1$ is hydrogen or a pharmacologically labile protecting group;

$R^2$ and $R^3$ are each independently hydrogen, alkyl, aryl, alkylaryl, or $COOR^4$ wherein $R^4$ is hydrogen, lower alkyl, lower alkenyl, aryl or arylalkyl, or a pharmacologically labile protecting group with the proviso that one of $R^2$ and $R^3$ must be $-COOR^4$; and the other of $R^2$ and $R^3$ is hydrogen, alkyl, aryl or alkylaryl;

$R^5$ is hydrogen or hydroxy when $R^3$ is $COOR^4$ and $R^5$ is hydrogen, alkyl, aryl or arylalkyl when $R^2$ is $COOR^4$;

$R^5$ and $R^7$ are each independently hydrogen, hydroxy, alkoxy, alkyl, aryl, arylalkyl, arylalkyloxy, halogen, trifluoromethyl or, when taken together, in the case of phenyl, with the ring carbons to which they are attached form a carbocyclic ring;

B is phenyl or a heteroaryl ring such as thiophene, furan or pyridine; when B is a 5-membered ring such as thiophene or furan then ring substitution is restricted by the number of available ring carbon atoms. Therefore, in this case only one substituent is possible. In this case $R^6$ may be H, alkyl, OH or OR.

X is $-(CH_2)_n-$, $-C=C-$, $-OCH_2-$, $-OCH_2CH_2-$, $-SCH_2-$, $-SOCH_2$, $-SO_2CH_2-$, $-NHCH_2-$, $-(CRR)_n$, $-(CH_2)_nCHR-$, $-CH_2CHRCH_2-$, $-CHR(CH_2)_n-$, $-CRR(CH_2)_n-$, $-(CH_2)_nCRR-$, $-CH_2CRRCH_2-$, $-CHRCHRCH_2-$, $-CH_2CHRCHR$, $-CHRCH_2CHR-$, n is an integer of from 0 to 2, R is hydrogen, hydroxyl or alkyl and only one R on a carbon atom is hydroxy, and R and R may form a ring.

A is $-OPO_3R^8R^9$, $-PO_3R^8R^9$, $-PO_2R^8R^8$, $-BO_2R^8R^9$, $-CO_2R^8$ or $-SO_3R^8$ or tetrazole wherein $R^8$ and $R^9$ are each independently hydrogen, alkyl, alkenyl, aryl, arylalkyl or a pharmaceutically acceptable labile group.

Preferred compounds of the instant invention as those of formula I wherein

$R^2$ and $R^3$ are each independently hydrogen, alkyl or $COOR^4$ wherein $R^4$ is hydrogen, lower alkyl, lower alkenyl with the proviso that one of $R^2$ and $R^3$ must be $COOR^4$ and the other of $R^2$ and $R^3$ is hydrogen or lower alkyl;

$R^6$ and $R^7$ are each independently hydrogen, hydroxy, lower alkyl, aryl, or, in the case of phenyl, are taken together with the ring carbon to which they are attached to form $-CH=CH-CH=CH-$;

X is $-C=C-$, $-CRR(CH_2)_n-$, $-(CH_2)_nCRR-$, $-CH_2CRRCH_2-$, $-(CH_2)_n-$ wherein n is an integer of from 0 to 2;

A is $-PO_3R^8R^9$, $-PO_2R^8R^9$, $COOR^8$, or tetrazole wherein $R^8$ and $R^9$ are each independently hydrogen, lower alkyl or a pharmaceutically acceptable labile group; and

B is phenyl, thiophene or furan.

More preferred compounds of the instant invention are those of formula I wherein

$R^2$ and $R^3$ are each independently hydrogen, lower alkyl or $COOR^4$ wherein $R^4$ is hydrogen or lower alkyl with the same proviso as above;

$R^5$ is hydrogen or hydroxy when $R^3$ is $COOR^4$ or $R^5$ is hydrogen or alkyl when $R^2$ is $COOR^4$;

$R^5$ and $R^7$ are each independently hydrogen, hydroxyl lower alkyl or when taken together with the ring to which they are attached form $-CH=CH-CH=CH-$;

A is $PO_3R^8R^9$, $COOR^8$ or tetrazole; and
B is phenyl or thiophene.

Still more preferred compounds of the instant invention are those of formula I above wherein:
$R^1$ is hydrogen;
$R^2$ and $R^3$ are each independently hydrogen or COOH with the above proviso that one of $R^2$ and $R^3$ must be $COOR^4$ and the other of $R^2$ and $R^3$ is hydrogen;
$R^5$ is hydrogen;
$R^5$ and $R^7$ are hydrogen or when taken together, in the case of phenyl, with the ring carbons to which they are attached, form -CH = CH-CH = CH-;
X is $(CH_2)_n$ wherein n is 0 to 2; and
A is $PO_3R^8R^9$ or tetrazole wherein $R^8$ and $R^9$ are each hydrogen or a pharmaceutically acceptable labile group.

Yet more preferred compounds of the instant invention include but are not limited to
1,2,3,4-tetrahydro-5-(1H-tetrazol-5-ylmethyl)-3-isoquinolinecarboxlic acid,
1,2,3,4-tetrahydro-5-(1H-tetrazol-5-yl))-3-isoquinolinecarboxylic acid,
1,2,3,4-tetrahydro-7-(1H-tetrazol-5-yl)-1-isoquinolinecarboxylic acid,
4,5,6,7-tetrahydro-3-(2-phosphonoethyl)thieno[2,3-c]pyridine-5-carboxylic acid,
4,5,6,7-tetrahydro-3-[2-(1H-tetrazol-5-yl)ethyl]thieno[2,3-c]pyridine-5-carboxylic acid,
4,5,6,7-tetrahydro-2-phosphonothieno[2,3-c]pyridine-7-carboxylic acid, and
4,5,6,7-tetrahydro-2-(1H-tetrazol-5-yl)thieno[2,3-c]pyridine-7-carboxylic acid.

The most preferred compounds of the present invention include but are not limited to
1,2,3,4-tetrahydro-5-phosphono-3-isoquinolinecarboxylic acid,
1,2,3,4-tetrahydro-5-(phosphonomethyl)-3-isoquinolinecarboxylic acid,
1,2,3,4-tetrahydro-5-(2-phosphonoethyl)-3-isoquinolinecarboxylic acid,
1,2,3,4-tetrahydro-7-phosphono-1-isoquinolinecarboxylic acid,
1,2,3,4-tetrahydro-5-(2-phosphonoethyl)benz[g]isoquinoline-3-carboxylic acid, and
1,2,3,4-tetrahydro-5-[2-(1H-tetrazol-5-yl)ethyl]-3-isoquinolinecarboxylic acid.

Novel intermediates useful in the preparation of compounds of formula I are those of formulae below.
Dimethyl 5-bromo-3,4-dihydro-2,3(1H)-isoquinolinedicarboxylate,
Dimethyl 7-bromo-3,4-dihydro-2,3(1H)-isoquinolinedicarboxylate,
Dimethyl 5-(diethoxyphosphinyl)-3,4-dihydro-2,3(1H)-isoquinolinedicarboxylate,
Dimethyl 7-(diethoxyphosphinyl)-3,4-dihydro-2,3(1H)-isoquinolinedicarboxylate,
Dimethyl 5-[2-(diethoxyphosphinyl)ethenyl]-3,4-dihydro-2,3(1H)-isoquinolinedicarboxylate,
Dimethyl 7-[2-(diethoxyphosphinyl)ethenyl]-3,4-dihydro-2,3(1H)-isoquinolinedicarboxylate,
Dimethyl 5-[2-(diethoxyphosphinyl)ethyl]-3,4-dihydro-2,3(1H)-isoquinolinedicarboxylate,
Dimethyl 7-[2-(diethoxyphosphinyl)ethyl]-3,4-dihydro-2,3(1H)-isoquinolinedicarboxylate,
Triethyl 1,4-dihydro-5-[(diethoxyphosphinyl)methyl]-2,3,3-isoquinolinetricarboxylate,
Triethyl 1,4-dihydro-7-[(diethoxyphosphinyl)methyl]-2,3,3-isoquinolinetricarboxylate,
7-Bromo-3,4-dihydro-1,2(1H)-isoquinolinedicarboxylic acid, 2-ethyl ester,
2-Ethyl-1-methyl-7-bromo-3,4-dihydro-1,2(1H)isoquinolinedicarboxylate,
2-Ethyl-1-methyl-7-(diethoxyphosphinyl)-3,4-dihydro-1,2(1H)-isoquinolinedicarboxylate,
Diethyl [[3-(2-aminoethyl)phenyl]methyl]phosphonate,
Diethyl [[4-(2-aminoethyl)phenyl]methyl]phosphonate,
Ethyl [2-[3-(diethoxyphosphinyl)phenyl]ethyl]carbamate,
Ethyl [2-[4-(diethoxyphosphinyl)phenyl]ethyl]carbamate,
6-[(Diethoxyphosphinyl)methyl]-3,4-dihydro-1,2(1H)-isoquinolinedicarboxylic acid, 2-ethyl ester,
7-[(Diethoxyphosphinyl)methyl]-3,4-dihydro-1,2(1H)-isoquinolinedicarboxylic acid, 2-ethyl ester,
Diethyl 6-[(diethoxyphosphinyl)methyl]-3,4-dihydro-1,2(1H)-isoquinolinedicarboxylate,
2-Ethyl-1-methyl-7-[(diethoxyphosphinyl)methyl]-3,4-dihydro-1,2(1H)-isoquinolinedicarboxylate,
Diethyl 5-isoquinolinylphosphonate, N-oxide,
Diethyl 7-isoquinolinylphosphonate, N-oxide,
Diethyl [2(7-isoquinolinyl)ethyl]phosphonate, N-oxide,
Diethyl (1-cyano-5-isoquinolinyl)phosphonate,
Diethyl (1-cyano-7-isoquinolinyl)phosphonate,
Diethyl [2-(1-cyano-7-isoquinolinyl)ethyl]phosphonate,
Diethyl [1-(aminocarbonyl)-5-isoquinolinyl]phosphonate,
Diethyl [1-(aminocarbonyl)-7-isoquinolinyl]phosphonate,
Diethyl [2-[1-(aminocarbonyl)-7-isoquinolinyl]ethyl]phosphonate,

Diethyl [1-(aminocarbonyl)-1,2,3,4-tetrahydro-5-isoquinolinyl]phosphonate,
Diethyl [1-(aminocarbonyl)-1,2,3,4-tetrahydro-7-isoquinolinyl]phosphonate,
Diethyl [2-[1-(aminocarbonyl)-1,2,3,4-tetrahydro-7-isoquinolinyl]ethyl]phosphonate,
Diethyl [[1-[2-(diethoxyphosphinyl)ethenyl]-Z-naphthalenyl]methyl][(ethoxycarbonyl)amino]propanedioate,
Diethyl [[1-[2-(diethoxyphosphinyl)ethyl]-Z-naphthalenyl]methyl] [(ethoxycarbonyl)amino]propanedioate,
Triethyl 5-[2-(diethoxyphosphinyl)ethyl]-1,4-dihydrobenz[g]isoquinoline-2,3,3-tricarboxylate,
Diethyl [[2-(2-cyanoethyl)phenyl]methyl][(ethoxycarbonyl)amino]propanedioate,
Triethyl 5-(2-cyanoethyl)-1,4-dihydro-2,3,3-isoquinolinetricarboxylate, and
Triethyl 1,4-dihydro-5-[2-(1H-tetrazol-5-yl)ethyl] -2,3,3-isoquinolinetricarboxylate.

The compounds of the instant invention include all solvates, hydrates, and salts of a compound of formula I above.

The term alkyl refers to a straight or branched chain of from one to six carbon atoms including but not limited to methyl, ethyl, n-propyl, isopropyl, propyl, n-butyl, sec-butyl, isobutyl, tert-butyl, n-pentyl, n-hexyl, and the like. The preferred alkyl groups are methyl and ethyl.

The term aryl includes but is not limited to phenyl, thienyl, naphthyl, and the like. Phenyl is the preferred aryl. The aryl group may be unsubstituted or substituted by one to three substituents selected from lower alkyl, lower alkoxy, trifluoromethyl or halogen.

The term arylalkyl means an aryl as described above attached to an alkyl as described above. This includes but is not limited to benzyl, 2-phenylethyl or 3-phenylpropyl.

The term alkenyl means a straight or branched unsaturated carbon chain of from two to six carbon atoms. This includes but is not limited to 2-propenyl, 1-butenyl, 2-butenyl, 1-pentenyl, 2-pentenyl, 3-methyl-3-butenyl, 1-hexenyl, and the like.

The term halogen means fluorine, chlorine, bromine, or iodine. The preferred halogens are fluorine and chlorine.

The term pharmaceutically acceptable labile group is a group that is readily removed under physiological conditions. In a compound of formula I above, A is $-OPO_3R^8R^9$, $-PO_3R^8R^9$, $-PO_2R^8R^8$, $-BO_2R^8R^9$, $-CO_2R^8$, or $-SO_3R^8$ tetrazole, $R^8$ and $R^9$ may be a pharmaceutically acceptable labile group such as amino substituted lower alkyl; mono or di lower alkyl amino substituted lower alkyl; carboxy substituted lower alkyl, e.g., α-carboxy substituted lower alkyl; lower alkoxy carbonyl substituted lower alkyl, e.g., α-lower alkoxycarbonyl substituted lower alkyl; pyridylmethyl; lower alkanoyloxy substituted methyl, e.g., pivaloyloxymethyl; lower alkanoyloxy or lower alkoxy substituted lower alkoxymethyl; bicyclo[2.2.1]heptyloxycarbonyl substituted methyl, e.g., bornyloxycarbonylmethyl; 3-phthalido; lower alkyl, lower alkoxy or halo substituted 3-phthalido; or lower alkoxycarbonyloxy lower alkyl; e.g., 1-methoxy or 1-ethoxycarbonyloxyethyl. Preferably $R^8$ and $R^9$ may be a pharmaceutically acceptable labile group such as lower alkanoyloxy substituted methyl, e.g., pivaloyloxymethyl-3-phthalido; di lower alkyl amino alkyl, e.g., 2-diethyl aminoethyl; or pyridylmethyl, e.g., 3-pyridylmethyl.

Pharmaceutically labile groups include but are not limited to such derivatives as are described by
I. H. Pitman in Med Chem Rev 2:189 (1981);
J. Alexander, R. Cargill, S. R. Michaelson, and H. Schwan in J Med Chem 31:318 (1988).

Salts of the compounds of the invention are preferably pharmaceutically acceptable salts. The compounds of the invention having a free phosphonic or carboxy group, more particularly alkali or alkaline earth metal salts, e.g., the sodium, potassium, magnesium or calcium salt; or advantageously crystallizing ammonium salts derived from ammonia or organic amines, such as methylamine, diethylamine, triethylamine, dicyclohexylamine, triethanolamine, ethylenediamine, tris-(hydroxymethyl)aminomethane or benzyltrimethylammonium. On the other hand, the compounds of the invention which are basic amines form acid addition salts of preferably pharmaceutically acceptable inorganic or organic acids, such as of strong mineral acids, for example, hydrohalic, e.g., hydrochloric or hydrobromic acid; sulfuric, phosphoric or nitric acid; aliphatic or aromatic carboxylic or sulfonic acids, e.g., acetic, propionic, succinic, glycolic, lactic, malic, tartaric, gluconic, citric, ascorbic, maleic, fumaric, pyruvic, pamoic, nicotinic, methanesulfonic, ethanesulfonic, hydroxyethanesulfonic, benzenesulfonic, p-toluenesulfonic or naphthalenesulfonic acid.

For isolation or purification purposes, salts may be obtained which might not be useful for pharmaceutical purposes. However, only pharmaceutically acceptable salts are used for therapeutic purposes and these salts, therefore, are preferred.

The compounds of the present invention may contain asymmetric carbon atoms. The instant invention includes the individual enantiomers, diastereomers, or mixtures thereof which may be prepared or isolated by methods known in the art.

The compounds of the instant invention exhibit valuable pharmacological properties by selectively blocking the N-methyl-D-aspartate sensitive excitatory amino acid receptors in mammals. The compounds

are thus useful for treating diseases responsive to excitatory amino acid blockade in mammals.

The effects are demonstrable in in vitro tests or in vivo animal tests using mammals or tissues or enzyme preparations thereof, e.g., from mice, rats, or monkeys. The compounds are administered enterally or parenterally, for example, orally, transdermally, subcutaneously, intravenously or intraperitoneally. Forms include but are not limited to gelatin capsules or agueous suspensions or solutions. The applied in vivo dosage may range between about 0.01 to 100 mg/kg, preferably between about 0.05 and 50 mg/kg, most preferably between about 0.1 and 10 mg/kg.

The ability of the compounds of the instant invention to antagonize the action of the endogenous ligand at the N-methyl-d-aspartate receptor is assessed by their ability to displace $[^3H]$-CPP from a biochemical receptor preparation. This preparation is described in J Pharmacol Exp Ther , 238, 739-749 (1986). Examples I, III, V, IX, X, XI bind to this receptor with an $IC_{50}$ of less than 100 micromolar.

The present invention also includes processes for making the compounds of formula I above.

One process for the preparation of compounds of formula I wherein $R^3$ is COOH, X is $(CH_2)_n$ and n is 2 is illustrated in Scheme A below.

Step (1) Reacting a compound of the formula X

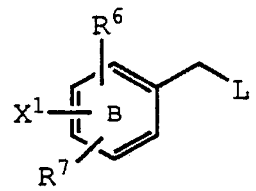

X

the above ring B, which is formula X is phenyl or heteroaryl, L is a leaving group such as methanesulfonate, toluenesulfonate, Cl, Br, or I, wherein $X^1$ is Br, I, $OSO_2CF_3$, and $R^5$ and $R^7$ are as defined above with diethylacetamidomalonate, dimethylacetamidomalonate, diethylformamidomalonate or the like, in the presence of sodium methoxide or sodium ethoxide in a solvent such as methanol or ethanol or the like, over a period of 24 hours at room temperature to obtain a compound of the formula XI,

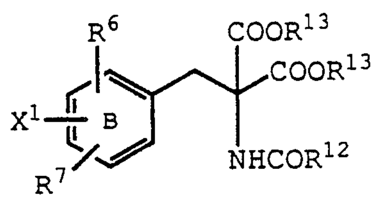

XI

wherein $R^{12}$ is lower alkyl, aryl, arylalkyl, and $R^{13}$ is lower alkyl or arylalkyl.

Step (2) The compounds of the formula XI are then treated under aqueous acid conditions, for example, aqueous 6N HCl over a period of 24 to 48 hours at temperature from 80 °C to reflux to obtain a compound of the formula XII.

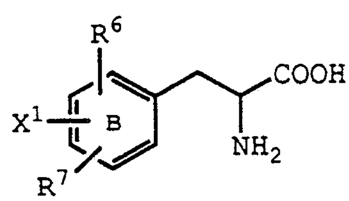

XII

Step (3) The compounds of the formula XII are then treated with ethylchloroformate, methylchloroformate, benzylchloroformate, or the like at room temperature in a solvent such as aqueous 1N NaOH, agueous IN KOH or the like over a period of 1 to 24 hours to obtain a compound of the formula XIII.

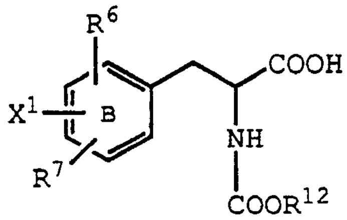

XIII

Step (4) The compounds of the formula XIII are then treated with an esterifying reagent such as diazomethane at room temperatures in a solvent such as diethyl ether, tetrahydrofuran or the like (or by other methods known to those who are skilled in the art) to obtain a compound of the formula XIV

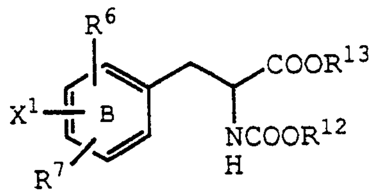

XIV

wherein $R^{13}$ is lower alkyl, aryl, or arylalkyl.

Step (5) The compounds of the formula XIV are then treated with an aldehyde such as paraformaldehyde, 1,3,5-trioxane acetaldehyde, benzaldehyde, or the like in a solvent such as acetic acid, sulfuric acid, trifluoroacetic acid, or combinations thereof or the like to obtain a compound of the formula XV.

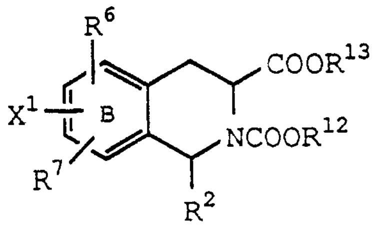

XV

Step (6) The compound of formula XV is then treated with a compound of the formula (XVI)

$H_2C=CHD$ XVI

wherein D is CN, $CO_2R^{12}$, or $PO(OR^1{}_a)(OR^2{}_b)$ and $R^1{}_a$ and $R^2{}_b$ are lower alkyl, or other protecting groups known to those skilled in the art, in the presence of bis(triphenylphosphine)palladium dichloride or the like and triethylamine or tributylamine or the like in a solvent such as toluene, dimethylformamide or the like to obtain the compound of the formula XVII

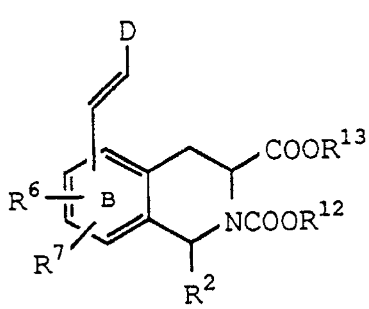

XVII

Step (7) The compound of the formula XVII is hydrogenated using 10% palladium on carbon or the like in a solvent such as methanol, ethanol or the like under a hydrogen atmosphere to give the compound of the formula XVIII

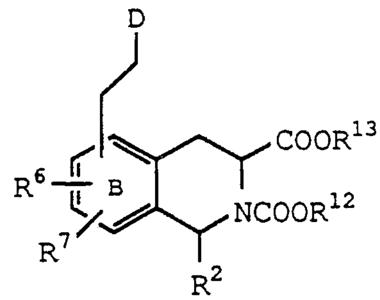

XVIII

Step (8) A compound of the formula XVIII wherein D is $PO(OR^1{}_a)(OR^2{}_b)$ is then hydrolyzed by treatment with aqueous 6N HCl, aqueous 6N $H_2SO_4$ or the like at reflux over a period of 10-72 hours or by other methods known to those who are skilled in the art to obtain a compound of formula I wherein X is $(CH_2)_n$, n is 2 and $R^3$ is COOH, and A is $PO_3H_2$.

Step (9) Alternatively, a compound of formula XVIII wherein D is CN, is treated with sodium azide and ammonium chloride or triethylamine hydrochloride or the like in a solvent such as N-methylpyrrolidinone, DMF or the like at an elevated temperature, for example, about 120° C, or by other methods known to those skilled in the art, to afford a compound of the formula XIX.

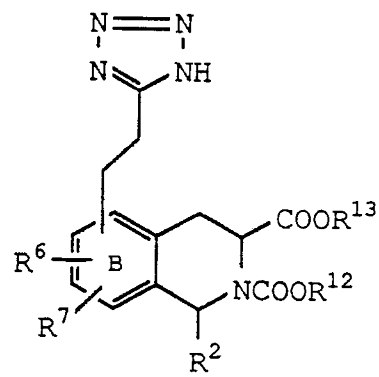

XIX

Step (10) A compound of the formula XIX is then hydrolyzed using aqueous 6N HCl, or aqueous 6N $H_2SO_4$ or the like at reflux or by other methods known to those who are skilled in the art to give the compound of the formula I wherein X is $(CH_2)_n$, n is 2, $R^3$ is COOH, and A is tetrazole.

SCHEME A

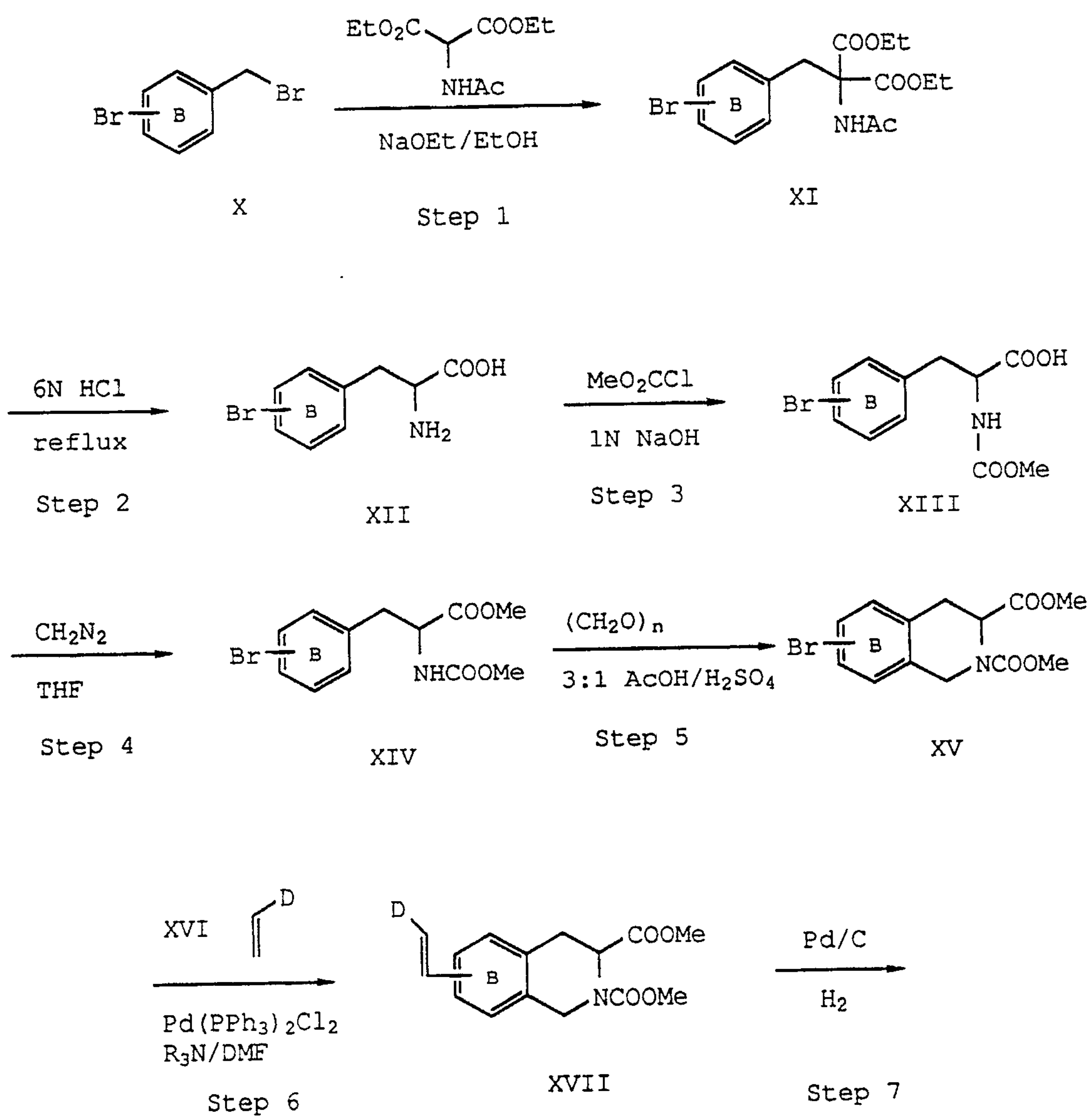
NaOEt/EtOH
X
Step 1
XI
6N HCl
reflux
Step 2
XII
$MeO_2CCl$
1N NaOH
Step 3
XIII
$CH_2N_2$
THF
Step 4
XIV
$(CH_2O)_n$
3:1 $AcOH/H_2SO_4$
Step 5
XV
XVI
$Pd(PPh_3)_2Cl_2$
$R_3N/DMF$
Step 6
XVII
Pd/C
$H_2$
Step 7

SCHEME A
(Continued)

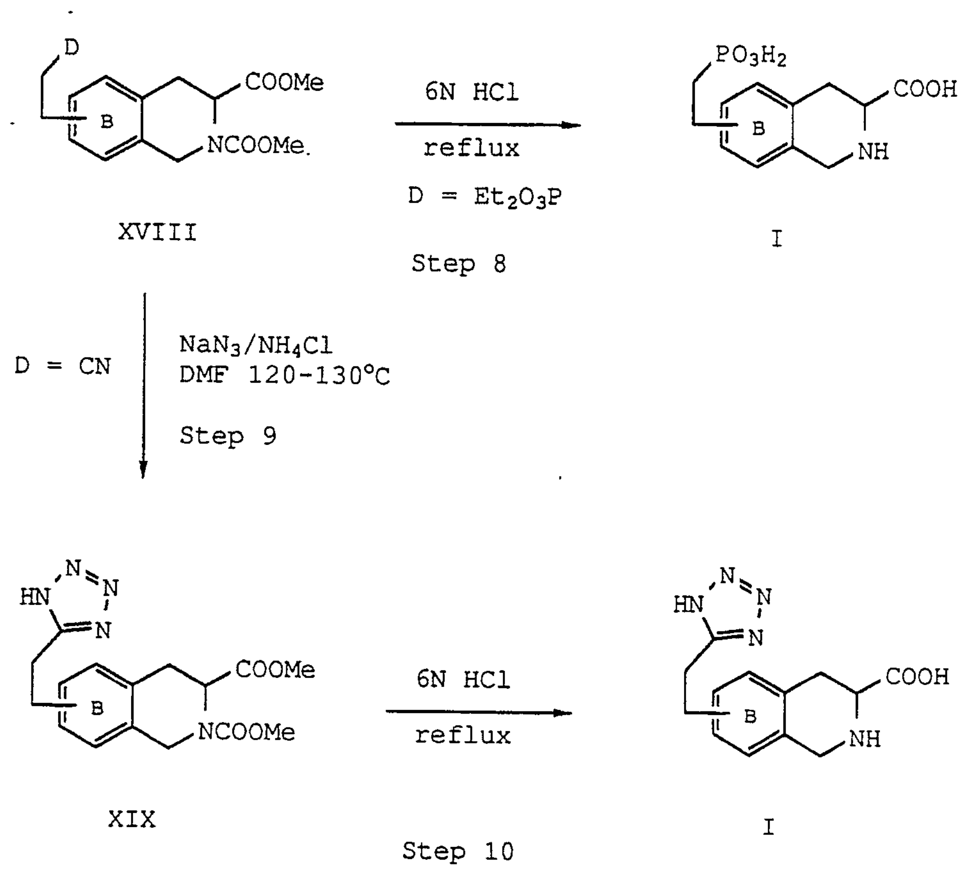


XVIII I

XIX I

In another process of the present invention the compounds of the formula I wherein $R^2$ = H, $R^3$ = COOH, X is $(CH_2)_n$, and n is 0 is prepared in a process, as illustrated by Scheme B below. This comprises

Step (1) Reaction of the compound of formula XV with $HPO(OR^1{}_a)(OR^2{}_b)$ in a palladium exchange of the $X^1$ substituent using conditions found in Synthesis 56-57 (1981), to give the compound of formula XX

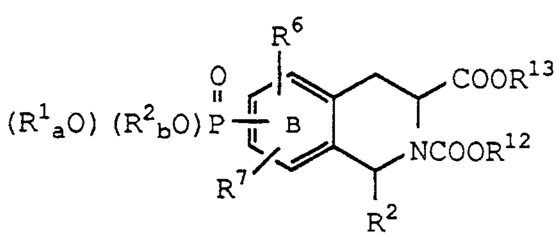

XX

Step (2) The compound of the formula XX is hydrolyzed by treatment with aqueous 6N HCl or aqueous 6N $H_2SO_4$ or the like or by other methods known to those who are skilled in the art to obtain the compounds of formula I wherein X is $(CH_2)_n$, n is 0, $R^2$ is H, $R^3$ is COOH, and A is $PO_3H_2$.

Step (3) Alternatively, the compound of formula XV is reacted with copper cyanide in a solvent such as DMF, quinoline or the like (or by other methods known to those who are skilled in the art such as those described in Chem Rev 779-794 (1982)) to give the compound of formula XXI.

Alternatively, when A is $CO_2R$ then this may be prepared by treating a compound of the formula XV

with carbon monoxide and a lower alkanol such as methanol in the presence of a palladium catalyst using methodology similar to that described in J Org Chem 1974 , 3318.

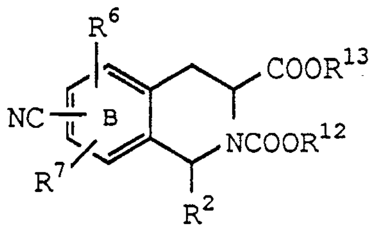

XXI

Step (4) The compound of the formula XXI is treated with sodium azide and ammonium chloride or triethylamine hydrochloride in a solvent such as N-methylpyrrolidinone or DMF at temperatures between 120 and 140 °C to give the compound of the formula XXII.

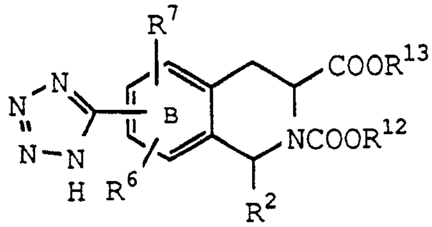

XXII

Step (5) The compound of formula XXII is then hydrolyzed to give a compound of formula I wherein X is $(CH_2)_n$, n is 0, $R^3$ is COOH, and A is tetrazole.

SCHEME B

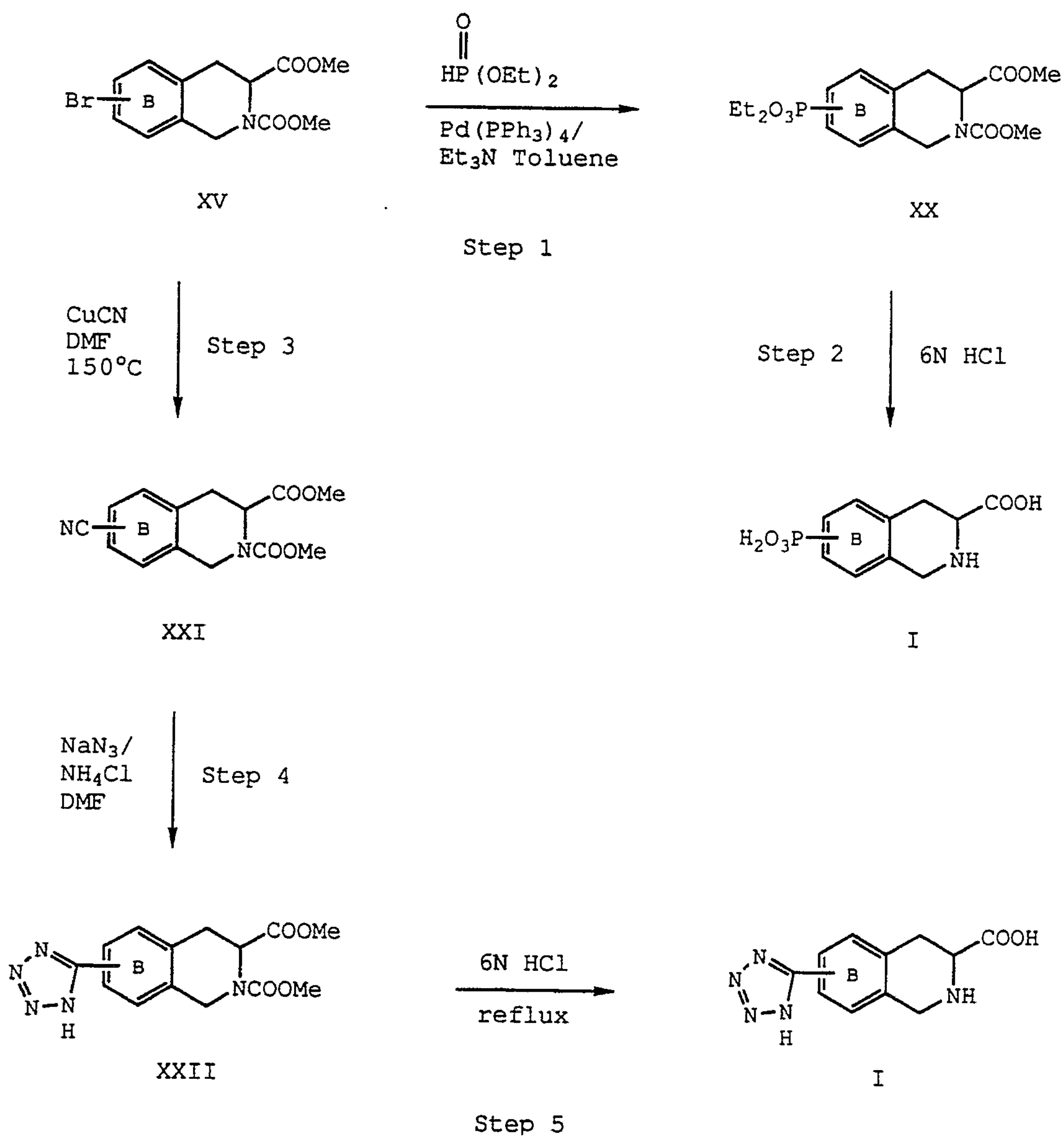


XV

XX

XXI

I

XXII

I

In another process of the present invention the compounds of formula I wherein X is $(CH_2)_n$, n is 1-2, and $R^3$ is COOH are prepared in a process, as illustrated by Scheme C below. This comprises

Step (1) Reaction of a compound of the formula XXIII

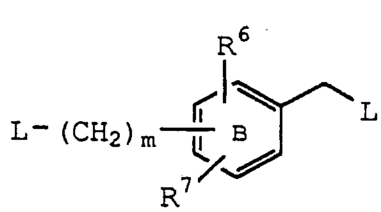

XXIII

wherein L, $R^6$, and $R^7$ are as previously defined and m is 1 with a compound of the formula

$R^{10}D$

wherein $R^{10}$ is sodium, potassium or the like and D is $PO(OR^1a)(OR^2b)$ or CN in a solvent such as diethyl ether, tetrahydrofuran, DMF or DMSO or the like to give a compound of the formula XXIV

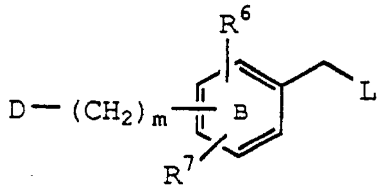

XXIV

Step (2) Sodium ethoxide in ethanol or the like is reacted with N-carboethoxydiethylaminomalonate or the like and the compound of the formula XXIV to obtain a compound of the formula XXV

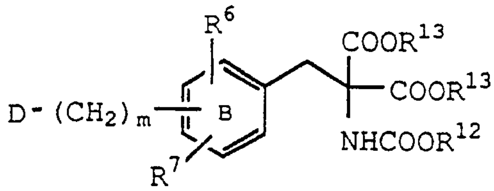

XXV

Step (3) Alternatively, the compound of the formula XXIII wherein m is 2, is treated with N-carboethoxydiethylaminomalonate or the like in the presence of sodium ethoxide or the like in ethanol or the like to afford the compound of the formula XXVI.

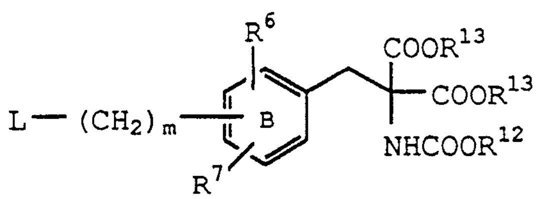

XXVI

Step (4) The compound of the formula XXVI wherein m is 1 or 2 is then treated with a compound of the formula $R^{10}D$ wherein $R^{10}$ and D are as previously defined in a solvent such as THF, ether, dimethylformamide, dimethylsulfoxide, or the like to afford a compound of the formula XXV.

Alternatively, the compound of the formula XXVI wherein m is 0 is treated with a compound of the formula XVI

$H_2C = CHD$ XVI

in the presence of bis(triphenylphosphine)palladium, dichloride or the like and triethylamine, tributylamine or the like in a solvent such as toluene, dimethylformamide or the like to obtain the compound of the formula XXVII.

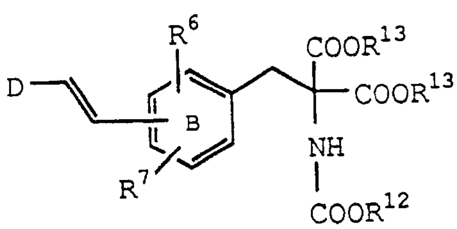

XXVII

The compound of the formula XXVII is hydrogenated using 10% palladium on carbon or the like in a solvent such as ethanol, methanol or the like to give the compound of formula XXV.

Step (5) The compound of the formula XXV is then treated with an aldehyde, such as paraformaldehyde,

acetaldehyde, benzaldehyde or the like at room temperature for a period of 1-48 hours to obtain a compound of the formula XXVIII.

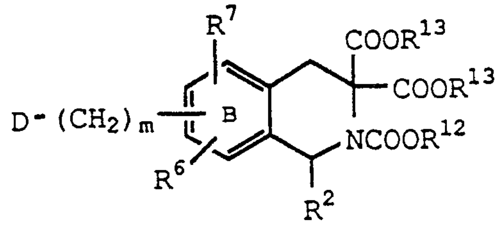

XXVIII

Step (6) The compounds of the formula XXVIII wherein D is $PO(OR^1{}_a)(OR^2{}_b)$ is hydrolyzed by treatment with aqueous 6N HCl, aqueous 6N $H_2SO_4$ or the like at reflux over a period of 10-72 hours to obtain the compound of formula I wherein X is $(CH_2)_n$, n is 1-2, and $R^3$ is COOH and A is $PO_3H_2$.

Step (7) Alternatively, the compound of formula XXVIII wherein D is CN, is treated with sodium azide and ammonium chloride or triethylamine hydrochloride or the like in a solvent such as N-methylpyrrolidinone, DMF or the like at a temperature of 100-180 °C to obtain the compound of the formula XXIX.

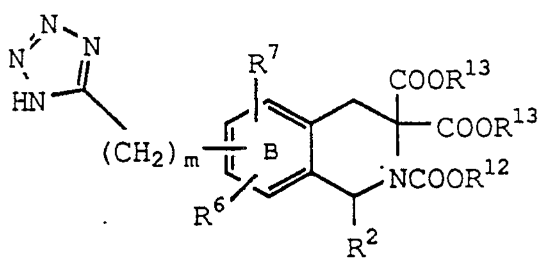

XXIX

Step (8) The compound of the formula XXIX is then hydrolyzed using aqueous 6N HCl, aqueous 6N $H_2SO_4$ or the like to give the compounds of formula I wherein X is $(CH_2)_n$, n is 1-2, $R^3$ is COOH, and A is tetrazole.

SCHEME C

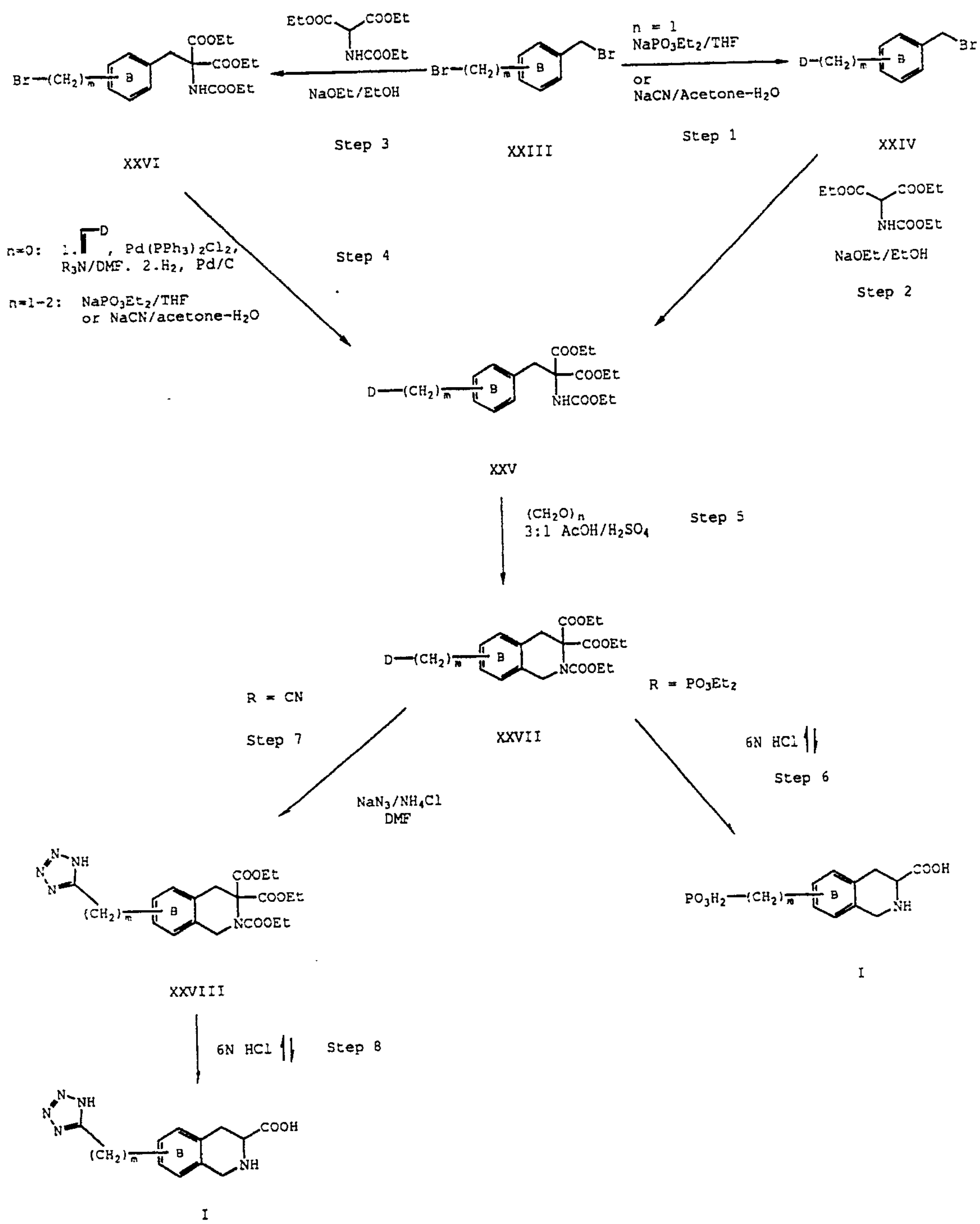


In another process of the present invention the compounds of the formula I wherein X is $(CH_2)_n$, n is 0 and 2 and $R^2$ is COOH is illustrated in Scheme D below. This comprises

Step (1) Reaction of a compound of the formula XXX

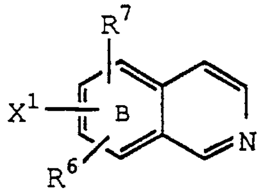

XXX

wherein $X^1$ is $OSO_2CF_3$, Br, I with a compound of formula $HPO(OR^1a)(OR^2b)$ in the presence of a palladium catalyst as described in Synthesis 56-57 (1981) or J Amer Chem Soc 109(9) : 2381 (1987) to obtain a compound of the formula XXXI

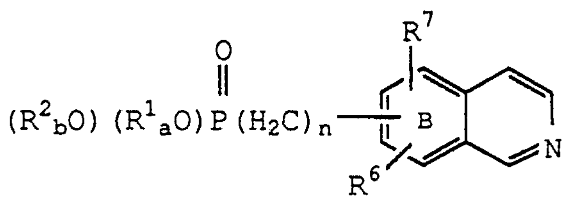

XXXI

wherein n is 0.

Step (2) Alternatively the compound of formula XXX is treated with a compound of formula

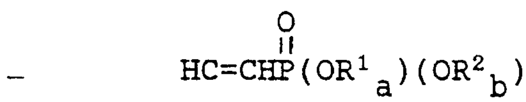

in the presence of bis(triphenylphosphine)palladium dichloride or the like and triethylamine, tributylamine or the like in a solvent such as toluene, xylene, or dimethylformamide or the like at a temperature between 80-130 °C to obtain the compounds of the formula XXXIa.

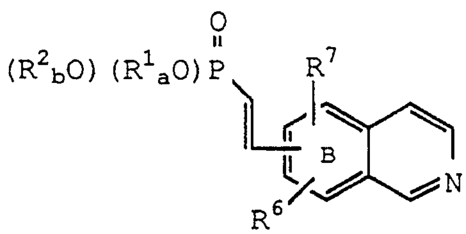

XXX1a

Step (3) The compound of formula XXX1a is hydrogenated to give a compound of the formula XXXI wherein n is 2.

Step (4) The compounds of the formula XXXI are treated with m-chloroperoxybenzoic acid or the like in a solvent such as dichloromethane, chloroform or the like at 0 °C to room temperature to afford the compounds of the formula XXXII.

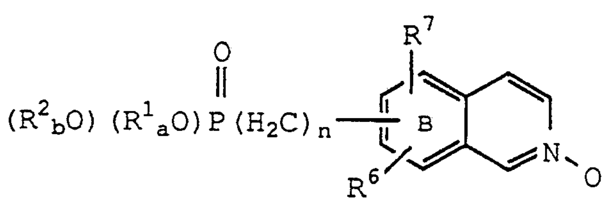

XXXII

Step (5) The compounds of the formula XXXII are then treated with trimethylsilyl cyanide or the like in a solvent such as triethylamine, tributylamine or the like at temperatures between 90-120 °C over a period of 24 hours to give the compounds of the formula XXXIII.

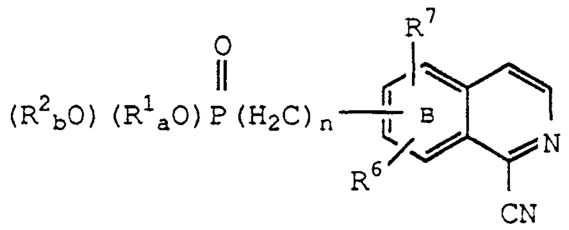

XXXIII

Step (6) The compounds of the formula XXXIII are then treated with an acid such as sulfuric acid at 90° C for a period of 5-30 minutes to give the compounds of the formula XXXIV.

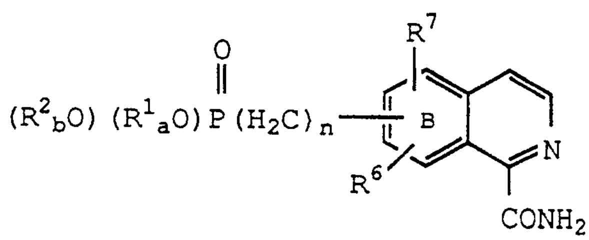

XXXIV

Step (7) The compounds of the formula XXXIV are then hydrogenated to obtain the compounds of the formula XXXV.

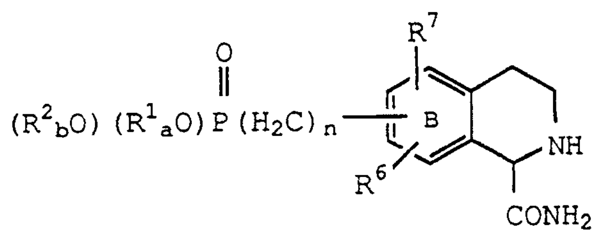

XXXV

Step (8) The compounds of the formula XXXV are then hydrolyzed to obtain the compounds of formula I wherein X is $(CH_2)_n$, n is 0 or 2, $R^2$ is COOH, and A is $PO_3H_2$.

SCHEME D

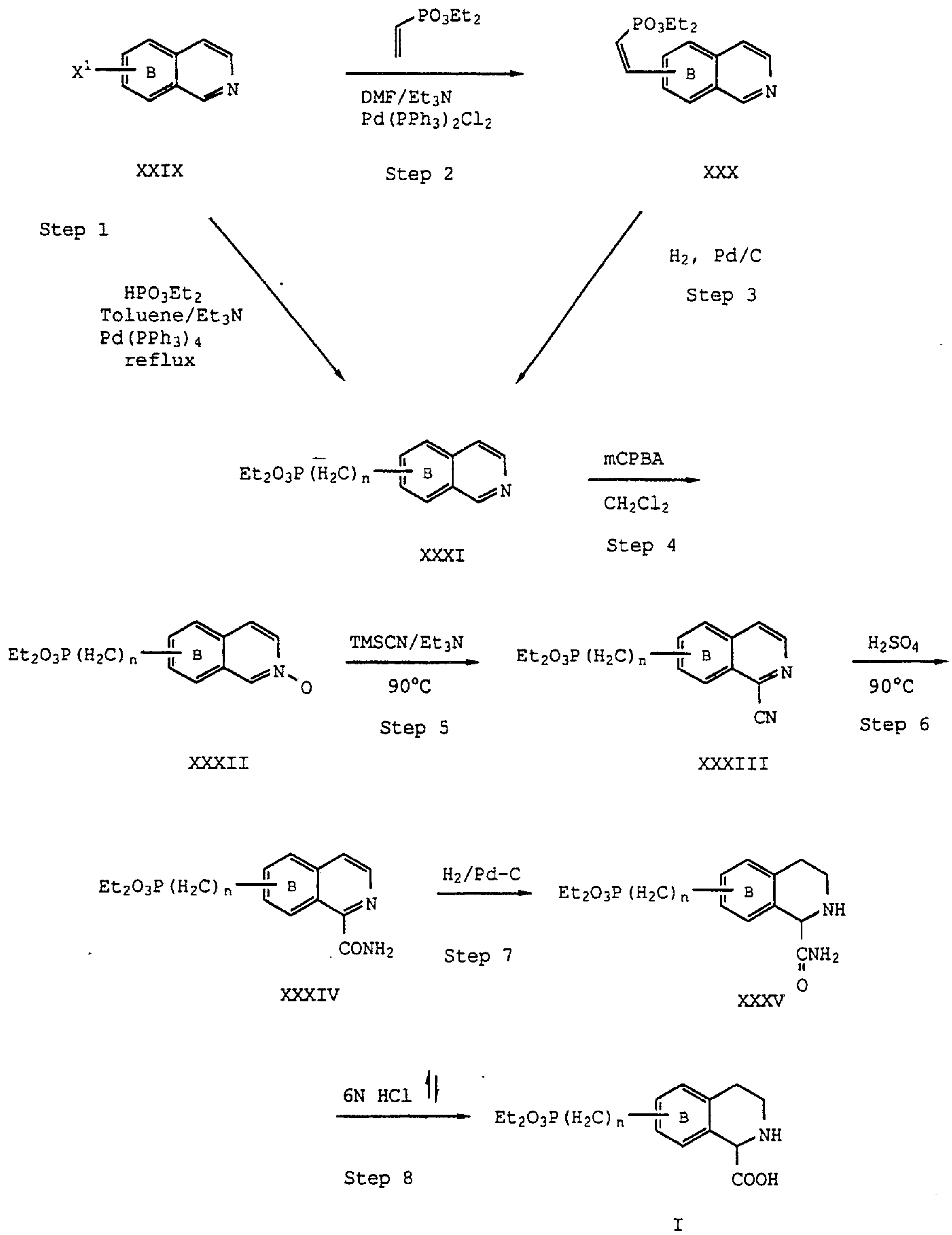


In another process of the present invention the compounds of the formula I wherein $R^2$ is COOH, and X is $(CH_2)_n$, and n is 0 is prepared in a process as illustrated by Scheme E below. This comprises

Step (1) Reacting a compound of the formula XXXVI

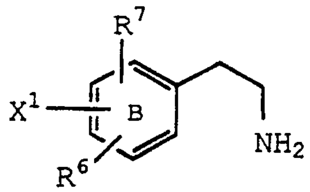

XXXVI

with ethylchloroformate, methylchloroformate or the like in a solvent such as dichloromethane, chloroform or the like in the presence of a base such as triethylamine, tributylamine or the like over a period of 30 minutes to 24 hours at 0° C to obtain a compound of the formula XXXVII.

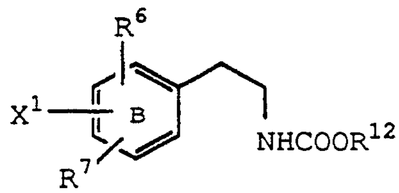

XXXVII

Step (2) The compounds of the formula XXXVII are then reacted with glyoxylic acid or ester thereof under similar conditions as described in Tetrahedron 43, 439 (1987) for compounds where aromatic substitution is not present, to obtain a compound of the formula XXXVIII.

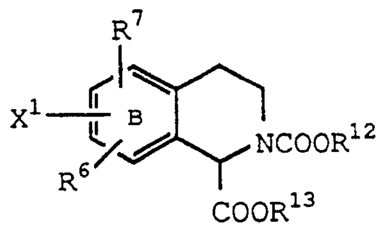

XXXVIII

Step (3) For ease of purification the compounds of the formula XXXVIII wherein $R^{13}$ is H may then be treated with an esterifying reagent such as diazomethane in a solvent such as tetrahydrofuran, ethyl ether or the like (or by other methods known to those who are skilled in the art) to obtain a compound of the formula XXXIX

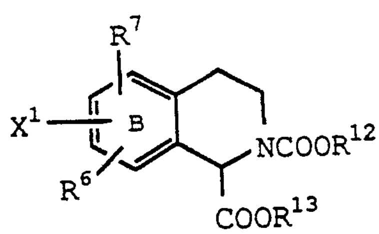

XXXIX

Step (4) The compounds of the formula XXXIX are then treated with $HPO(OR^1a)(OR^2b)$ in a palladium exchange of the $X^1$ substituent using conditions found in Synthesis 56-57 (1981) to afford the compounds of the formula XL.

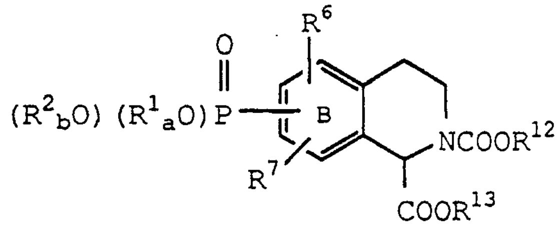

XL

Step (5) The compounds of the formula XL are hydrolyzed to afford the compounds of formula I wherein $R^2$ is COOH, X is $(CH_2)_n$, n is 0, and A is $PO_3H_2$.
Step (6) Alternatively, the compounds of the formula XXXIX are reacted with copper cyanide in a solvent such as DMF, quinoline, or the like (or by other methods known to those who are skilled in the art such as those described in Chem Rev 779-794 (1987)) to give the compounds of the formula XLI.

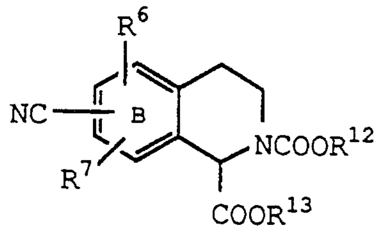

XLI

Step (7) The compounds of the formula XLI are then reacted with sodium azide and ammonium chloride, triethylamine hydrochloride or the like in a solvent such as DMF or the like at a temperature between 120-150 °C or by other methods known to those skilled in the art, to obtain the compounds of the formula XLII.

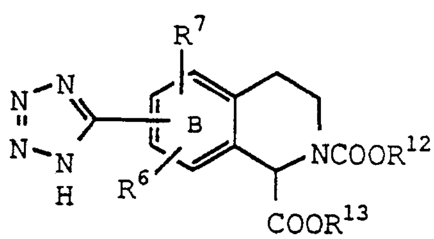

XLII

Step (8) The compounds of the formula XLII are then hydrolyzed to give the compounds of the formula I wherein $R^2$ is COOH, $R^3$ is H, and A is tetrazole.

SCHEME E

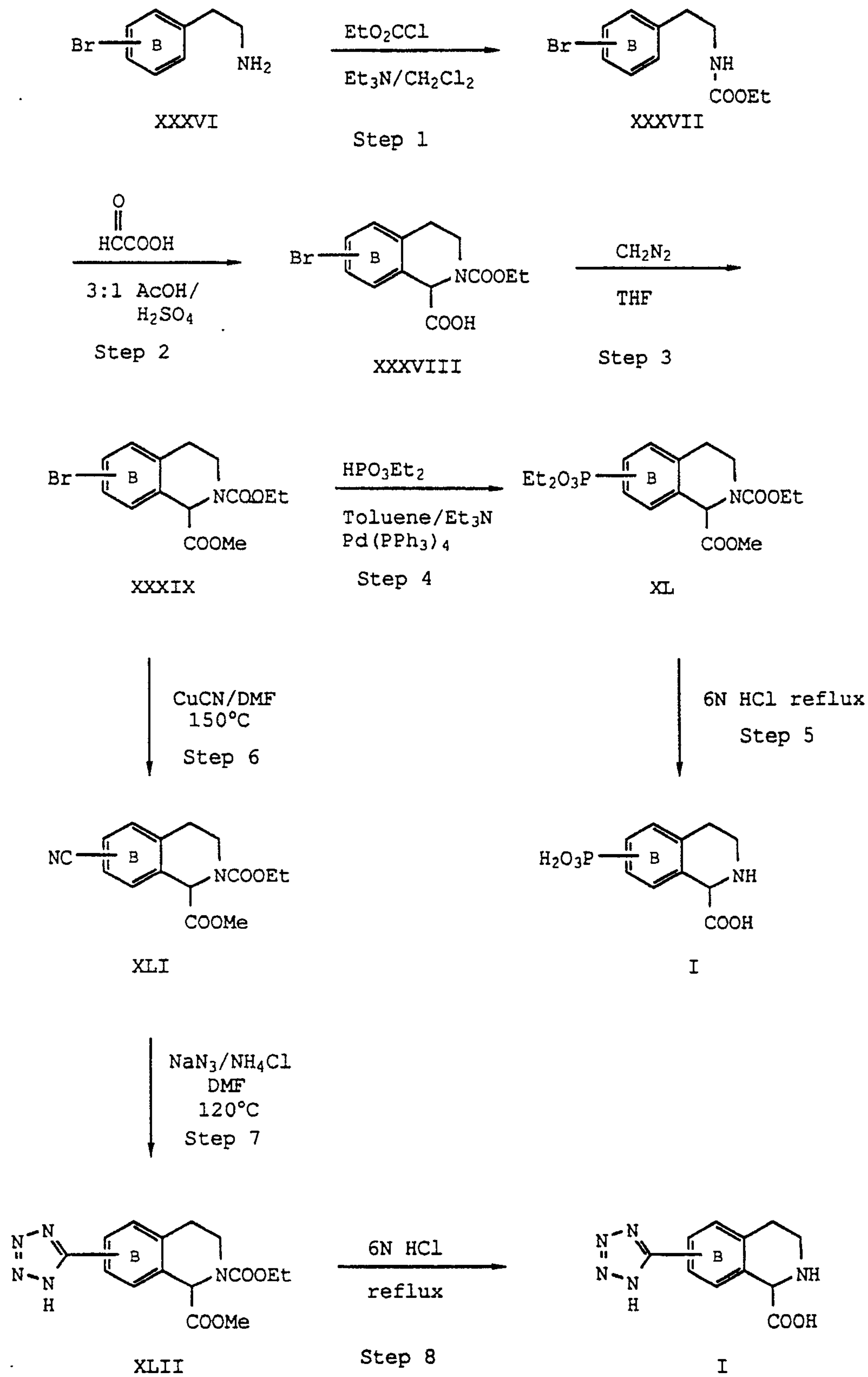


XXXVI XXXVII XXXVIII XXXIX XL XLI I XLII I

In another process of the present invention the compounds of the formula I wherein $R^2$ is COOH, X is $(CH_2)_n$, n is 1, and A is $PO_3H_2$ is prepared in a process as illustrated by Scheme F. This comprises

Step (1) Reaction of a compound of the formula XXVI

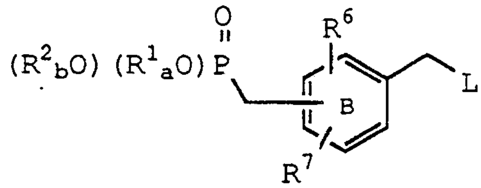

XXVI

with sodium cyanide or potassium cyanide in a solvent such as acetone, dioxane, water, or combination thereof to afford a compound of the formula XLIII.

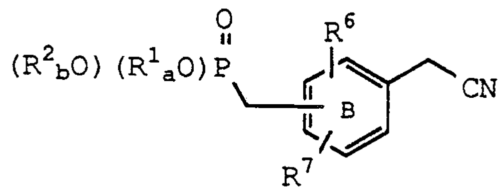

XLIII

Step (2) The compound of formula XLIII is then treated with zinc in methanolic ammonia (or by other reductive methods known to those who are skilled in the art) to give a compound of the formula XLIV.

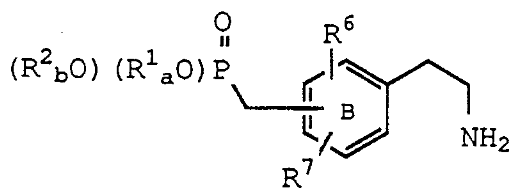

XLIV

Step (3) The compound of the formula XLIV is reacted with ethylchloroformate, methylchloroformate or the like in a solvent such as dichloromethane or chloroform or the like in the presence of a base such as triethylamine, tributylamine or the like over a period of 30 minutes to 24 hours at 0 °C to obtain a compound of the formula XLV wherein $R^{12}$ is as previously defined.

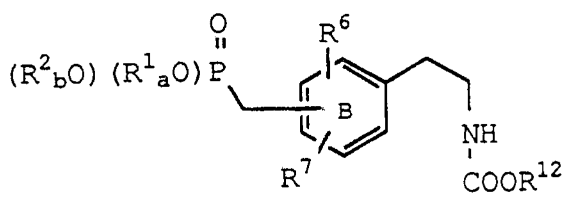

XLV

Step (4) The compound of the formula XLV is reacted with glyoxylic acid or ester thereof in a solvent such as acetic acid, sulfuric acid, trifluoroacetic acid or combinations thereof or the like to obtain a compound of the formula XLVI.

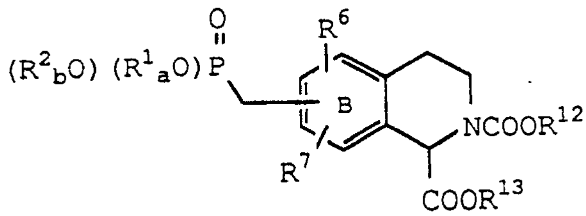

XLVI

Step (5) For ease of purification, the compound of formula XLVI wherein $R^{13}$ is H is treated with an esterifying reagent such as diazomethane in a solvent such as THF, ethyl ether, or the like to obtain a compound of formula XLVI wherein $R^{13}$ is lower alkyl.

Step (6) The compound of the formula XLVI is then hydrolyzed using 6N HCl or 6N $H_2SO_4$ or the like at reflux to give the compound of the formula I wherein X is $(CH_2)_n$, n is 1, and $R^2$ is COOH, and A is $PO_3H_2$.

SCHEME F

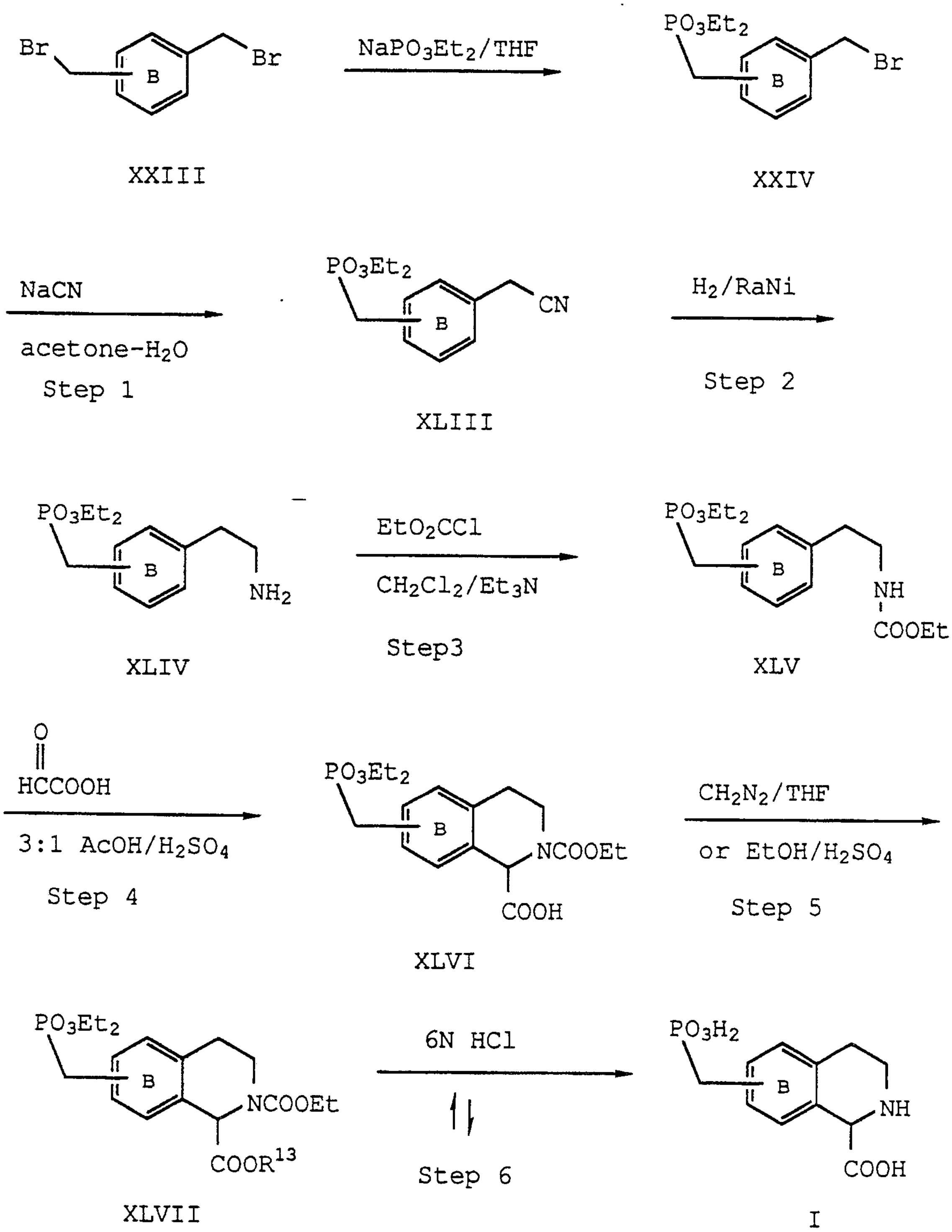


XXIII XXIV XLIII XLIV XLV XLVI XLVII I

The following examples are illustrative of the present invention but are not intended to limit it in any way. Preparations I-XXXIV are precursors and Examples I-XIII are final products.

Preparation I

Diethyl (acetylamino) [(2-bromophenyl)methyl]propanedioate.

Sodium (9.42 g, 0.40 mol) is dissolved in 800 ml of ethanol. The resulting solution is then treated with diethyl acetamidomalonate (87.2 g, 0.40 mol) and allowed to stir at room temperature overnight. A solution of 2-bromobenzylbromide (100 g, 0.40 mol; Aldrich) in 100 ml ethanol is added dropwise and the reaction is allowed to stir for 48 hours at room temperature. The reaction mixture is concentrated and the residue is treated with ethyl acetate (200 ml) and water (200 ml). The aqueous phase is extracted with ethyl acetate (3 x 200 ml) and the combined organic extracts are dried ($MgSO_4$), concentrated, and the residue recrystallized from hot 1:1 diisopropyl ether/heptane (900 ml). A white solid was obtained (114.1 g, 74%).

Preparation Ia

Diethyl (acetylamino)[(4-bromophenyl)methyl]propanedioate.

The corresponding compound of formula XI is prepared from 4-bromobenzyl bromide in a process analogous to Preparation I.

Preparation Ib

Diethyl [(1-bromo-2-naphthalenyl)methyl][(ethoxycarbonyl) amino] propanedioate.

The corresponding compound of formula XXV is prepared from 2-bromomethyl-1-bromonaphthalene and N-carboethoxydiethylaminomalonate in a process analogous to Preparation I.

Preparation II

2-Bromophenylalanine.

A suspension of diethyl(acetylamino)[(2-bromophenyl)methyl]propanedioate (110.2 g, 0.285 mol) in 1000 ml of aqueous 6N HCl is heated at reflux for 18 hours. The resulting solid is collected by suction filtration and dried under vacuum. A white solid is obtained (HCl salt, 71.5 g, 89%).

Preparation IIa

4-Bromophenylalanine.

The corresponding compound of formula XII is prepared from diethyl(acetylamino)[(4-bromophenyl)-methyl]propanedioate by a process analogous to Preparation II.

Preparation III

2-Bromo-N-(methoxycarbonyl)phenylalanine.

A solution of 2-bromophenylalanine (35.0 g, 0.125 mol) in 190 ml of aqueous 1N NaOH solution is treated dropwise with methyl chloroformate (18 ml, 0.232 mol). Additional aqueous 1N NaOH is added as

necessary to maintain a pH of 10. The resulting solution is extracted with ether (2 x 200 ml). The aqueous phase is acidified (pH = 2) with concentrated hydrochloric acid and extracted with dichloromethane (3 x 300 ml). The combined organic extracts are dried $Na_2SO_4$ and concentrated. A white solid is obtained (26.6 g, 71%).

Preparation IIIa

4-Bromo-N-(methoxycarbonyl)phenylalanine

The corresponding compound of formula XIII is prepared from 4-bromophenylalanine by a process analogous to Preparation III.

Preparation IV

Methyl 2-bromo-N-(methoxycarbonyl)phenylalaninate.

A solution of 2-bromo-N-(methoxycarbonyl)phenylalanine (26.6 g, 88.0 mmol) in 100 ml of tetrahydrofuran is treated with an ethereal solution of diazomethane until a persistent yellow color develops. The resulting solution is concentrated. An oil is obtained (26.6 g, 96%).

Preparation IVa

Methyl 4-bromo-N-(methoxycarbonyl)phenylalaninate.

The corresponding compound of formula XIV is prepared from 4-bromo-N-(methoxycarbonyl)-phenylalanine by a process analogous to Preparation IV.

Preparation V

Dimethyl 5-bromo-3,4-dihydro-2,3(1H)-isoquinolinedicarboxylate.

A solution of methyl 2-bromo-N-(methoxycarbonyl)phenylalaninate (14.7 g, 46.5 mmol) in 3:1 acetic acid/sulfuric acid (60 ml) is treated with paraformaldehyde (1.47 g, 49.0 mmol) and the resulting solution allowed to stir at room temperature for 24 hours. The reaction mixture is poured into water and extracted with ethyl acetate (4 x 50ml). The organic extracts are combined and washed with saturated aqueous $NaHCO_3$ solution, dried ($MgSO_4$), and concentrated. The residue is purified by silica gel chromatography (3:1 heptane/ethyl acetate). An oil is obtained (5.15 g, 33%).

Preparation Va

Dimethyl 7-bromo-3,4-dihydro-2,3(1H)-isoquinolinedicarboxylate.

The corresponding compound of formula XV is prepared from methyl 4-bromo-N-(methoxycarbonyl)-phenylalaninate by a process analogous to Preparation V.

Preparation VI

Dimethyl 5-(diethoxyphosphinyl)-3,4-dihydro-2,3(1H)-isoquinolinedicarboxylate.

A solution of dimethyl 5-bromo-3,4-dihydro-2,3(1H)-isoquinolinedicarboxylate (2.39 g, 7.28 mmol), diethylphosphite (1.11 g, 8.01 mmol), and tetrakis(triphenylphosphine)palladium (270 mg, 0.23 mmol) in toluene (20 ml) and triethylamine (1.15 ml) is heated to reflux under $N_2$ for 48 hours. The reaction mixture is cooled and washed with saturated aqueous $NaHCO_3$ solution. The aqueous phase is extracted with ethyl acetate. The combined organic extracts are dried ($MgSO_4$) and concentrated. The residue is purified by silica gel chromatography. An oil is obtained (1.54 g, 55%).

Preparation VIa

Dimethyl 7-(diethoxyphosphinyl)-3,4-dihydro-2,3(1H)-isoouinolinedicarboxylate.

The corresponding compound of formula XX is prepared from dimethyl 7-bromo-3,4-dihydro-2,3(1H)-isoquinolinedicarboxylate in a process analogous to Preparation VI.

Preparation VII

Dimethyl 5-[2-(diethoxyphosphinyl)ethenyl]-3,4-dihydro-2,3(1H)-isoquinolinedicarboxylate.

A solution of dimethyl 5-bromo-3,4-dihydro-2,3(1H)-isoquinolinedicarboxylate (4.50 g, 13.7 mmol) diethylvinylphosphonate (2.54 g, 15.5 mmol) and bis(triphenylphosphine)palladium dichloride (0.385 g, 0.55 mmol) in dimethylformamide (35 ml) and triethylamine (7 ml) is heated at 90° C for 24 hours. The reaction mixture is concentrated and the residue is purified by silica gel chromatography (ethyl acetate). A brown oil is obtained (2.26 g, 40%).

Preparation VIIa

Dimethyl 7-[2-(diethoxyphosphinyl)ethenyl]-3,4-dihydro-2,3(1H)isoquinolinedicarboxylate.

The corresponding compound of formula XVII is prepared from dimethyl 7-bromo-3,4-dihydro-2,3(1H)-isoquinolinedicarboxylate in a process analogous to Preparation VII.

Preparation VIII

Dimethyl 5-[2-(diethoxyphosphinyl)ethyl]-3,4-dihydro-2,3(1H)-isoquinolinedicarboxylate.

A solution of dimethyl 7-[2-(diethoxyphosphinyl)ethenyl]-3,4-dihydro-2,3(1H)-isoquinolinedicarboxylate (2.26 g, 5.49 mmol) in 100 ml methanol is hydrogenated at 52 psi over 10% palladium on carbon. The reaction mixture is filtered and concentrated. The residue is concentrated and purified by silica gel chromatography (ethyl acetate). An oil is obtained (1.44 g, 63%).

Preparation VIIIa

Dimethyl 7-[2-(diethoxyphosphinyl)ethyl]-3,4-dihydro-2,3(1H)-isoquinolinedicarboxylate.

The corresponding compound of formula XVIII is prepared from dimethyl 7-[2-(diethoxyphosphinyl)-ethenyl]-3,4-dihydro-2,3(1H)-isoquinolinedicarboxylate in a process analogous to Preparation VIII.

Preparation IX

Diethyl [[2-(bromomethyl)phenyl]methyl]phosphonate.

Sodium (2.89 g, 0.126 mol) is dissolved in a solution of diethylphosphite (17.4 g, 0.126 mol) and tetrahydrofuran (150 ml). The resulting solution is added at 0 °C to a stirring solution of o-dibromoxylene (100 g, 0.379 mol) in tetrahydrofuran (500 ml). The resulting solution is stirred for 48 hours at room temperature. The reaction mixture is treated with water (250 ml) and the phases separated. The organic phase is extracted with ethyl acetate (3 x 200 ml). The combined organic phases are dried ($MgSO_4$) and concentrated. The residue is purified by silica gel chromatography (heptane then ethyl acetate). A yellow oil is obtained (11.06 g, 27%).

Preparation IXa

Diethyl [[3-(bromomethyl)phenyl]methyl]phosphonate.

The corresponding compound of formula XXIV is prepared from m-dibromoxylene in a process analogous to Preparation IX.

Preparation IXb

Diethyl [[4-(bromomethylphenyl]methyl]phosphonate.

The corresponding compound of formula XXIV is prepared from p-dibromoxylene in a process analogous to Preparation IX.

Preparation X

Diethyl [[2-[(diethoxyphosphinyl)methyl]phenyl]methyl[(ethoxycarbonyl)amino] propanedioate.

Sodium (0.44 g, 19.1 mmol) is dissolved in 50 ml of ethanol. The resulting solution is treated with N-carboethoxydiethylaminomalonate (4.62 g, 18.7 mmol) in one portion and allowed to stir at room temperature for one hour. A solution of diethyl [[4-(bromomethyl) phenyl]methyl]phosphonate (6.00 g, 18.7 mmol) in ethanol (5 ml) is added dropwise and the resulting solution was stirred for 24 hours at room temperature. The reaction mixture was concentrated and the residue dissolved in ethyl acetate (100 ml) and water (100 ml). The organic layer is separated and the aqueous layer is extracted with ethyl acetate (3 x 100 ml). The combined organic extracts are dried ($MgSO_4$) and concentrated. The residue is purified by silica gel chromatography (ethyl acetate). An oil is obtained (6.91 g, 76%).

Preparation Xa

Diethyl [[4-[(diethoxyphosphinyl)methyl]phenyl]methyl][(ethoxycarbonyl)amino]propanedioate.

The corresponding compound of formula XXV is prepared from diethyl [[4-(bromomethyl)phenyl]-methyl]phosphonate in a process analogous to Preparation X.

Preparation Xb

Diethyl [[2(2-bromoethyl)phenyl]methyl][(ethoxycarbonyl)amino]propanedioate.

The corresponding compound of formula XXVI is prepared from 2-(2-bromoethyl)benzyl bromide (J Chem Soc 1950 , 1037) in a process analogous to Preparation X.

Preparation XI

Diethyl [[2-(2-cyanoethyl)phenyl]methyl][(ethoxycarbonyl)amino]propanedioate.

A solution of diethyl [[2-(2-bromoethyl)phenyl]methyl][(ethoxycarbonyl)amino]propanedioate (21.7 g, 48.8 mmol) and sodium cyanide (15.0 g, 0.305 mol) in 100 ml of dimethylsulfoxide is heated at 85° C under a nitrogen atmosphere. The reaction mixture is poured into 500 ml ice water and extracted with ether (3 x 150 ml). The combined organic extracts are washed with brine (2 x 75 ml), dried, filtered, and concentrated. A yellow oil is obtained (17.9 g, 97%).

Preparation XII

Diethyl [[1-[2-(diethoxyphosphinyl)ethenyl]-2-naphthalenyl]methyl][(ethoxycarbonyl)amino]propanedioate.

A solution of diethyl [(1-bromo-2-naphthalenyl)methyl][(ethoxycarbonyl)amino]propanedioate (7.66 g, 16.0 mmol) and diethylvinylphosphonate (2.54 ml, 16.4 mmol) and bis(triphenylphosphine)palladium dichloride (0.58 g, 0.82 mmol) in 5:1 dimethylformamide/triethylamine (100 ml) is heated at 90° C for 24 hours. The reaction mixture is concentrated and the residue purified by silica gel chromatography (1:1 heptane/ethyl acetate). An oil is obtained (3.17 g, 35%).

Preparation XIII

Diethyl [[1-[2-(diethoxyphosphinyl)ethyl]-2-naphthalenyl]methyl][(ethoxycarbonyl)amino]propanedioate.

A solution of diethyl [[1-[2-(diethoxyphosphinyl)ethenyl]-2-naphthalenyl]methyl][(ethoxycarbonyl)amino]-propanedioate (3.05 g, 5.43 mmol) in 100 ml of ethanol is hydrogenated at 52 psi over 5% palladium on carbon (0.6 g). The reaction mixture is concentrated. An oil is obtained (2.61 g, 85%).

Preparation XIV

Triethyl 1,4-dihydro-5-[(diethoxyphosphinyl)methyl]2,3,3-isoquinolinetricarboxylate.

A solution of diethyl [[2-[(diethoxyphosphinyl)methyl]phenyl]methyl] [(ethoxycarbonyl)amino]-propanedioate (4.43 g, 9.09 mmol) in 40 ml of 3:1 acetic acid/sulfuric acid was treated with paraformaldehyde (0.35 g, 11.7 mmol). The resulting solution is allowed to stir at room temperature for 24 hours. The reaction mixture is poured into water and extracted into dichloromethane (4 x 100 ml). The combined organic phases are washed with saturated $NaHCO_3$ (aq), dried, and concentrated. The residue is purified by silica gel chromatography (ethyl acetate). An oil is obtained (3.97 g, 87%).

Preparation XIVa

Triethyl 1,4-dihydro-7-[(diethoxyphosphinyl)methyl]-2,3,3-isoquinolinetricarboxylate .

The corresponding compound of formula XXVIII is prepared from diethyl [[4-[(diethoxyphosphinyl)-methyl]phenyl]methyl] [(ethoxycarbonyl)amino]propanedioate in a process analogous to Preparation XIV.

Preparation XIVb

Triethyl 5-[2-(diethoxyphosphinyl)ethyl]-1,4-dihydrobenz[g]isoquinoline-2,3,3-tricarboxylate .

The corresponding compound of formula XXVIII is prepared from diethyl [[1-[2-(diethoxyphosphinyl)-ethyl]-2-naphthalenyl]methyl][(ethoxycarbonyl)amino]propanedioate in a process analogous to Preparation XIV.

Preparation XIVc

Triethyl 5-(2-cyanoethyl)-1,4-dihydro-2,3,3-isoguinolinetricarboxylate .

The corresponding compound of formula XXVIII is prepared from diethyl [[2-(2-cyanoethyl)phenyl]-methyl] [(ethoxycarbonyl)amino]propanedioate in a process analogous to Preparation XIV.

Preparation XV

Triethyl (1,4-dihydro-5-[2-(1H-tetrazol-5-yl)ethyl]-2,3,3-isoquinolinetricarboxylate .

A 0.15M solution of triethyl 5-(2-cyanoethyl)-1,4-dihydro-2,3,3-isoquinoline tricarboxylate in N-methylpyrrolidinone is treated with sodium azide (4 eq.) and triethylamine hydrochloride (2 eq.). The reaction mixture is heated to 145° C. The product is obtained by pouring the reaction mixture into water and extraction into ethyl acetate followed by drying and concentration.

Preparation XVI

7-(Trifluoromethyl)sulfonyl]oxyisoquinoline .

A solution of 7-hydroxyisoquinoline (9.75 g, 67.2 mmol) and diisopropylethylamine (11.5 ml) in methanol (150 ml) is cooled to 0 ° C and bistrifluoromethanesulfonyl aniline (30.0 g, 84 mmol) is added. The resulting solution was stirred at room temperature for 18 hours. The reaction mixture was concentrated and purified by silica gel chromatography (3:2 heptane/ethyl acetate). A colorless oil is obtained (14.4 g, 77%).

Preparation XVII

Diethyl 5-isoquinolinylphosphonate .

A solution of 5-bromoisoquinoline (5.0 g, 24 mmol), diethylphosphite (3.4 ml, 26 mmol), triethylamine (3.7 ml, 26 mmol) and tetrakistriphenylphosphine palladium (1.4 g, 1.2 mmol) in 25 ml of toluene is heated at 50 ° C under a $N_2$ atmosphere for 3 hours. The temperature is increased to 90 ° C for an additional 15 hours. The reaction is cooled to room temperature and additional toluene is added. The resulting mixture was filtered and the filtrate was concentrated. The residue was purified by silica gel chromatography (ethyl acetate). A yellow oil is obtained (5.04 g, 79%).

| Anal. calcd. for $C_{13}H_{16}NPO_3$: | | | |
|---|---|---|---|
| | C, 58.87; | H, 6.08; | N, 5.58. |
| Found: | C, 58.95; | H, 6.11; | N, 5.15. |

Preparation XVIIa

Diethyl 7-isoquinolinylphosphonate .

The corresponding compound of formula XXXI is prepared from 7-[(trifluoromethyl)sulfonyl]-oxyisoquinoline in a process analogous to Preparation XVII.

Preparation XVIII

Diethyl [2-(7-isoquinolinyl)ethenyl]phosphonate .

A solution of 7-[(trifluoromethyl)sulfonyl]oxyisoquinoline (14.4 g, 51.9 mmol) vinyldiethylphosphite (11.5 ml, 74.8 mmol), triethylamine (29.0 ml, 0.208 mol) and bistriphenyl phosphine palladium dichloride (1.1 g, 1.57 mmol) in dimethylformamide (100 ml) is heated at 90 ° C under a $N_2$ atmosphere. The reaction mixture is concentrated and the residue is purified by silica gel chromatography (9:1 ethyl acetate/ethanol). A red oil is obtained (19.9 g). This material is used directly in Preparation XIX without further purification.

Preparation XIX

Diethyl [2-(7-isoquinolinyl)ethyl]phosphonate .

A solution of diethyl [2-(7-isoquinolinyl)ethenyl]phosphonate (18.0 g, 61.9 mmol) in 100 ml of ethanol is hydrogenated at 52 psi over 5% palladium on carbon (2.0 g) for 71 hours. The reaction mixture is filtered,

concentrated, and the resulting oil purified by silica gel chromatography (9:1 ethyl acetate/ethanol). The material obtained is a mixture of diethyl [2-(7-isoquinolinyl)ethenyl]phosphonate and diethyl [2-(7-isoquinolinyl)ethyl]phosphonate and is dissolved in 100 ml of ethanol and is hydrogenated at 53 psi over 10% palladium on carbon (3.0 g) for 5.6 hours. The reaction mixture is filtered and concentrated. A yellow oil is obtained (11.5 g, 63%).

Preparation XX

Diethyl 5-isoquinolinylphosphonate, N-oxide .

A solution of diethyl 5-isoquinolinylphosphonate (4.30 g, 16.2 mmol) in 100 ml dichloromethane is treated with m-chloroperoxybenzoic acid (MCPBA) (3.0 g, 17.4 mmol). After 30 minutes additional MCPBA (1.6 g, 9.27 mmol) is added. The reaction is stirred for 18 hours at room temperature. The reaction mixture is quenched with 20% agueous $K_2CO_3$ (100 ml), the organic phase is separated, dried ($Na_2SO_4$) and concentrated. A gold oil is obtained (4.57 g, 100%).

Preparation XXa

Diethyl 7-isoquinolinylphosphonate, N-oxide .

The corresponding compound of formula XXXII is prepared from diethyl 7-isoquinolinylphosphonate in a process analogous to Preparation XX.

Preparation XXb

Diethyl [2-(7-isoquinolinyl)ethyl]phosphonate, N-oxide .

The corresponding compound of formula XXXII is prepared from diethyl [2-(7-isoquinolinyl)ethyl]-phosphonate in a process analogous to Preparation XX.

Preparation XXI

Diethyl (1-cyano-5-isoquinolinyl)phosphonate .

A suspension of diethyl 5-isoquinolinylphosphonate, N-oxide (4.57 g, 16.2 mmol) in triethylamine (5.6 ml, 40.2 mmol) and trimethylsilylcyanide (12.0 ml, 89.9 mmol) is heated at 90° C for 2 hours. The dark mixture was poured into a cold solution of saturated aqueous $NaHCO_3$. The resulting mixture was stirred for 1 hour and extracted into dichloromethane. The organic extracts are dried ($Na_2SO_4$) and concentrated. The residue is purified by silica gel chromatography (ethyl acetate). An orange solid is obtained (2.77 g, 59%).

| Anal. calcd. for $C_{14}H_{15}N_2O_3P$: | | | |
|---|---|---|---|
| | C, 57.9; | H, 5.21; | N, 9.65. |
| Found: | C, 57.53; | H, 4.90; | N, 9.40. |

Preparation XXIa

Diethyl (1-cyano-7-isoquinolinyl)phosphonate.

The corresponding compound of formula XXXIII is prepared from diethyl 7-isoquinolinylphosphonate, N-oxide in a process analogous to Preparation XXI.

Preparation XXIb

Diethyl [2-(1-cyano-7-isoquinolinyl)ethyl]phosphonate.

The corresponding compound of formula XXXIII is prepared from diethyl [2-(7-isoquinolinyl)ethyl]-phosphonate, N-oxide in a process analogous to Preparation XXI.

Preparation XXII

Diethyl [1-(aminocarbonyl)-5-isoquinolinyl]phosphonate.

A suspension of diethyl (1-cyano-5-isoquinolinyl)phosphonate (2.4 g, 8.53 mmol) in concentrated sulfuric acid (15 ml) is heated at 90° C for 10 minutes. The resulting solution is cooled to 0° C and poured into a solution of $Na_2CO_3$ (30 g) in water (200 ml) at 0° C. The resulting mixture is made basic by the addition of solid $Na_2CO_3$, and extracted into dichloromethane. The organic phase is dried ($Na_2SO_4$), filtered, and concentrated. A tan solid is obtained (1.32 g, 50%), mp 128-129° C.

| Anal. calcd. for $C_{14}H_{17}N_2O_4P$: | | | |
|---|---|---|---|
| | C, 54.55; | H, 5.56; | N, 9.09. |
| Found: | C, 54.46; | H, 5.57; | N, 8.96. |

Preparation XXIIa

Diethyl [1-(aminocarbonyl)-7-isoquinolinyl]phosphonate.

The corresponding compound of formula XXXIV is prepared from diethyl (1-cyano-7-isoquinolinyl)-phosphonate in a process analogous to Preparation XXII.

Preparation XXIIb

Diethyl [2-[1-(aminocarbonyl)-7-isoquinolinyl]ethyl]phosphonate.

The corresponding compound of formula XXXIV is prepared from diethyl [2-(1-cyano-7-isoquinolinyl)-ethyl]phosphonate in a process analogous to Preparation XXII.

Preparation XXIII

Diethyl [1-(aminocarbonyl)-1,2,3,4-tetrahydro-5-isoquinolinyl]phosphonate .

A solution of diethyl [1-(aminocarbonyl)-5-isoquinolinyl]phosphonate (1.15 g, 3.73 mmol) in 100 ml methanol is hydrogenated at 53 psi over 10% palladium on carbon (1.0 g) for 4.8 hours. The reaction mixture is concentrated, and the residue is purified by silica gel chromatography (ethyl acetate). An oil is obtained (0.91 g, 78%).

Preparation XXIIIa

Diethyl [1-(aminocarbonyl)-1,2,3,4-tetrahydro-7-isoquinolinyl]phosphonate .

The corresponding compound of formula XXXV is prepared from diethyl [1-(aminocarbonyl)-7-isoquinolinyl]phosphonate in a process analogous to Preparation XXIII.

Preparation XXIIIb

Diethyl [2-[1-(aminocarbonyl)-1,2,3,4-tetrahydro-7-isoquinolinyl]ethyl]phosphonate .

The corresponding compound of formula XXXV is prepared from diethyl [2-[1-(aminocarbonyl)-7-isoquinolinyl]ethyl]phosphonate in a process analogous to Preparation XXIII.

Preparation XXIV

Ethyl [2-(4-bromophenyl)ethyl]carbamate .

A solution of 4-bromophenethylamine (10.0 g, 50 mmol) and triethylamine (5.3 g, 52.5 mmol) in dichloromethane (200 ml) at 0 °C is treated with ethyl chloroformate (5.69 g, 52.5 mmol). The resulting solution is stirred at 0 °C for one hour and washed with saturated aqueous $NaHCO_3$ solution (50 ml). The organic phase is dried ($MgSO_4$) and concentrated. An oil is obtained (12.3 g, 90%).

Preparation XXV

7-Bromo-3,4-dihydro-1,2(1H)-isoquinolinedicarboxylic acid, 2-ethyl ester .

A solution of ethyl [2-(4-bromophenyl)ethyl]carbamate (10.8 g, 39.8 mmol) in 3:1 acetic acid/sulfuric acid (80 ml) is treated with glyoxylic acid monohydrate (4.07 g, 44.2 mmol) and the resulting solution is allowed to stir at room temperature for 24 hours. The reaction mixture is poured onto ice (50 g) and the resulting aqueous layer is extracted with dichloromethane (5 x 50 ml). The combined organic extracts are dried ($Na_2SO_4$) and concentrated. A white solid is obtained (11.4 g, 87%).

Preparation XXVI

2-Ethyl-1-methyl 7-bromo-3,4-dihydro-1,2(1H)-isoquinolinedicarboxylate .

A solution of 7-bromo-3,4-dihydro-1,2(1H)-isoquinolinedicarboxylic acid, 2-ethyl ester (8.35 g, 25.4 mmol) in tetrahydrofuran (100 ml) is treated with an ethereal solution of diazomethane until a persistent yellow color develops. The reaction mixture is concentrated. The resulting solid is suspended in a 5:1 heptane/diisopropyl ether solution and the resulting suspension filtered. A white solid is obtained (4.09 g, 47%). The filtrate is concentrated to afford a less pure fraction (4.08 g, 46%).

Preparation XXVII

1-Ethyl-1-methyl 7-(diethoxyphosphinyl)-3,4-dihydro-1,2(1H)-isoquinolindicarboxylate .

A solution of 2-ethyl-1-methyl 7-bromo-3,4-dihydro-1,2(1H)-isoquinolinedicarboxylate (4.00 g, 11.7 mmol), diethylphosphite (1.69 g, 12.3 mmol) tetrakis(triphenylphosphine)palladium (0.68 g, 0.6 mmol), and triethylamine (1.67 g, 16.5 mmol) in toluene (50 ml) is heated at reflux for 24 hours. The reaction mixture is cooled to room temperature and washed with water (20 ml). The aqueous phase is extracted with ethyl acetate (2 x 30 ml) and the combined organic extracts are dried ($MgSO_4$), and concentrated. The residue is purified by silica gel chromatography (ethyl acetate). A yellow oil is obtained (2.69 g, 58%).

Preparation XXVIII

Diethyl [[4-(cyanomethyl)phenyl]methyl]phosphonate .

A mixture of diethyl [[4-(bromomethyl)phenyl]methyl]phosphonate (15.0 g, 47 mmol), potassium cyanide (4.6 g, 70 mmol), and sodium iodide (0.5 g, 3.3 mmol) in acetone (50 ml) and water (15 ml) at 50 °C is stirred vigorously for 48 hours. The mixture is diluted with water and extracted with dichloromethane. The organic layer is washed with water, dried ($Na_2SO_4$) and concentrated. A gold liquid is obtained (13.0 g, quantitative).

Preparation XXVIIIa

Diethyl [[3-(cyanomethylphenyl]methyl]phosphonate .

The corresponding compound of formula XLIII is prepared from diethyl [[3-(bromomethyl)phenyl]-methyl]phosphonate in a process analogous to Preparation XXVIII.

Preparation XXIX

Diethyl [[3-(2-aminoethyl)phenyl]methyl]phosphonate .

A suspension of sodium borohydride (1.62 g, 42.8 mmol) in tetrahydrofuran (20 ml) is treated with trifluoroacetic acid (3.2 ml, 41.5 mmol) in tetrahydrofuran (5 ml). The resulting solution is treated with diethyl [[3-(cyanomethyl)phenyl]methyl]phosphonate (11.21 g, 41.9 mmol) in tetrahydrofuran (20 ml), is allowed to

stir overnight at room temperature. The reaction mixture is treated with water (15 ml) and stirred overnight. The reaction mixture is concentrated and the residue is taken up in dichloromethane, washed with water, dried ($Na_2SO_4$), and concentrated. A yellow oil is obtained (8.93 g, 77%).

### Preparation XXX

Diethyl [[4-(2-aminoethyl)phenyl]methyl]phosphonate .

A solution of diethyl [[4-(cyanomethyl)phenyl]methyl]phosphonate (3.4 g, 12.7 mmol) in a methanol-ammonia solution is hydrogenated over Raney nickel. The resulting solution is filtered and concentrated. A pale green oil is obtained (3.26 g, 95%).

### Preparation XXXI

Ethyl [2-[3-(diethoxyphosphinyl)phenyl]ethyl]carbamate .

A solution of diethyl [[3-(2-aminoethyl)phenyl]methyl]phosphonate (1.44 g, 5.29 mmol) and triethylamine (0.82 ml, 5.82 mmol) in dichloromethane (10 ml) is cooled to 0° C and treated with ethyl chloroformate (0.56 ml, 5.82 mmol) in dichloromethane (10 ml). The resulting solution is stirred overnight at room temperature. The reaction mixture is diluted with dichloromethane (50 ml), washed with saturated aqueous $NaHCO_3$ (20 ml), dried ($Na_2SO_4$), and concentrated. The residue is purified by silica gel chromatography (ethyl acetate). An oil is obtained (1.37 g, 75%).

### Preparation XXXIa

Ethyl [2-[4-(diethoxyphosphinyl)phenyl]ethyl]carbamate .

The corresponding compound of formula XLV is prepared from diethyl [[4(2-aminoethyl)phenyl]methyl]-phosphonate in a process analogous to Preparation XXXI.

### Preparation XXXII

6-[Diethoxyphosphinyl)methyl]-3,4-dihydro-1,2(1H)-isoquinolinedicarboxylic acid, 2-ethyl ester .

A solution of ethyl [2-[3-(diethoxyphosphinyl)phenyl]ethylcarbamate (0.69 g, 2.01 mmol) and glyoxylic acid monohydrate (0.17 g, 1.84 mmol) in 3:1 acetic acid/sulfuric acid (4 ml) is stirred at room temperature for 44 hours. The reaction mixture is poured into water (50 ml) and extracted into ethyl acetate (3 x 50 ml). The combined organic phases are dried ($Na_2SO_4$) and concentrated. A brown oil is obtained (0.64 g, 80%).

### Preparation XXXIIa

7-[(Diethoxyphosphinyl)methyl]-3,4-dihydro-1,2(1H)-isoquinolinedicarboxylic acid, 2-ethyl ester .

The corresponding compound of formula XLVI is prepared from ethyl [2-[4-(diethoxyphosphinyl)phenyl]-ethyl]carbamate in a process analogous to Preparation XXXII.

Preparation XXXIII

Diethyl 6-[(diethoxyphosphinyl)methyl]-3,4-dihydro-1,2(1H)-isoquinolinedicarboxylate .

A solution of 6-[(diethoxyphosphinyl)methyl]-3,4-dihydro-1,2(1H)-isoquinolinedicarboxylic acid, 2-ethyl ester (0.59 g, 1.48 mmol) in ethanol (9.5 ml) and concentrated sulfuric acid (0.5 ml) is heated at reflux for 24 hours. The reaction mixture is poured into water (50 ml) and extracted into dichloromethane (4 x 50 ml). The combined organic extracts are dried ($Na_2SO_4$) and concentrated. The residue is purified by silica gel chromatography (ethyl acetate). An oil is obtained (0.28 g, 44%).

Preparation XXXIV

2-Ethyl-1-methyl 7-(diethoxyphosphinyl)methyl]-3,4-dihydro-1,2(1H)-isoquinolinedicarboxylate .

A solution of 7-[(diethoxyphosphinyl)methyl]-3,4-dihydro-1,2(1H)-isoquinolinedicarboxylic acid, 2-ethyl ester (5.4 g, 13.5 mmol) in tetrahydrofuran is treated with a solution of diazomethane in ether until a persistent yellow color has formed. The reaction is quenched with acetic acid and concentrated. The residue is purified by silica gel chromatography (gradient-elution, 50-100% EtOAc in heptane). An oil is obtained (3.85 g, 69%).

Example I

1,2,3,4-Tetrahydro-5-phosphono-3-isoquinolinecarboxylic acid .

A solution of dimethyl 5-(diethoxyphosphinyl)-3,4-dihydro-2,3(1H)-isoquinolinedicarboxylate (1.56 g, 4.05 mmol) in 30 ml of 6N HCl is heated at reflux for 24 hours. The resulting solution is concentrated and the residue dried under vacuum. The residue is suspended in water and the solid collected and dried at 100 °C ($P_2O_5$). A white solid is obtained (0.59 g, 56%), mp 292-295 °C (dec.).
Anal. calcd. for $C_{10}H_{12}NO_5P \cdot 0.07HCl$:
C, 46.24; H, 4.69; N, 5.39; Cl, 0.96. Found: C, 45.86; R, 4.53; N, 5.20; Cl, 0.86.

Example II

1,2,3,4-Tetrahydro-7-phosphono-3-isoquinolinecarboxylic acid .

A solution of dimethyl 7-(diethoxyphosphinyl)-3,4-dihydro-2,3(1H)-isoquinolinedicarboxylate (2.01 g, 5.21 mmol) in 20 ml of 6N HCl is heated at reflux for 48 hours. The reaction mixture is cooled to room temperature and extracted with ether. The aqueous phase is decolorized with charcoal, filtered, and concentrated. The residue is dissolved in 20 ml $H_2O$ and freeze-dried. A white solid is obtained (0.92 g, 61%).

| Anal. calcd. for $C_{10}H_{12}NO_5P \cdot 0.9HCl$: | | | | |
|---|---|---|---|---|
| | C, 41.42; | H, 4.48; | N, 4.83; | Cl, 11.00. |
| Found: | C, 41.16; | H, 4.46; | N, 4.52; | Cl, 10.94. |

Example III

1,2,3,4-Tetrahydro-5-(2-phosphonoethyl)-3-isoquinolinecarboxylic acid .

A solution of dimethyl 5-[2-(diethoxyphosphinyl)ethyl]-3,4-dihydro-2,3(1H)-isoquinolinedicarboxylate (1.26 g, 3.05 mmol) in 6N HCl (20 ml) is heated at reflux for 24 hours. The reaction mixture is concentrated and the residue dissolved in water (25 ml) and freeze-dried. The residue is purified by ion exchange chromatography (Dowex 50x4-400 ion exchange resin), eluting with 2M $NH_4OH$ solution. The eluant is freeze-dried and the residue dried under vacuum at 100° C ($P_2O_5$). A tan solid is obtained (0.439 g, 44.5%), mp 215-240° C (decomposed with gas evolution).

| Anal. calcd. for $C_{12}H_{16}NO_5P \cdot 1.46NH_3 \cdot 0.68 H_2O$: | | | |
|---|---|---|---|
| | C, 44.71; | H, 6.80; | N, 10.69. |
| Found: | C, 44.72; | H, 6.30; | N, 10.68. |

Example IV

1,2,3,4-Tetrahydro-7-(2-phosphonoethyl)-3-isoquinolinecarboxylic acid .

The corresponding compound of formula I is prepared from dimethyl 7-[2-(diethoxyphosphinyl)-ethyl]-3,4-dihydro-2,3(1H)-isoquinolinedicarboxylate by a procedure analogous to Example I.

| Anal. calcd. for $C_{12}H_{16}NO_5P \cdot 0.135HCl$: | | | | |
|---|---|---|---|---|
| | C, 49.67; | H, 5.61; | N, 4.83; | Cl, 1.65. |
| Found: | C, 50.27; | H, 5.41; | N, 4.61; | Cl, 1.65. |

Example V

1,2,3,4-Tetrahydro-5-(phosphonomethyl)-3-isoquinolinecarboxylic acid .

A mixture of triethyl 1,4-dihydro-5-[(diethoxyphosphinyl)methyl]-2,3,3-isoquinolinetricarboxylate (2.00 g, 4.02 mmol) in aqueous 6N HCl (50 ml) is heated at reflux for 48 hours. The reaction mixture is concentrated and the residue dissolved in 20 ml water and freeze-dried. The residue is dried under vacuum at 100° C. A white solid is obtained (1.06 g, 84%), mp 160-170° C (foamed).

| Anal. calcd. for $C_{11}H_{14}NO_5P \cdot 1.1HCl \cdot 0.2H_2O$: | | | | |
|---|---|---|---|---|
| | C, 41.96; | H, 4.96; | N, 4.45; | Cl, 12.38. |
| Found: | C, 42.07; | H, 4.91; | N, 4.30; | Cl, 12.17. |

Example VI

1,2,3,4-Tetrahydro-7-(phosphonomethyl)-3-isoquinolinecarboxylic acid .

The corresponding compound of formula I is prepared from triethyl 1,4-dihydro-7-[(diethoxyphosphinyl)-methyl]-2,3,3-isoquinolinetricarboxylate in a process analogous to Example V.

| Anal. calcd. for $C_{11}H_{14}NO_5P \cdot 0.75HCl \cdot 0.35H_2O$: | | | | |
|---|---|---|---|---|
| | C, 43.34; | H, 5.11; | N, 4.60; | Cl, 8.72. |
| Found: | C, 43.32; | H, 4.69; | N, 4.28; | Cl, 8.87. |

Example VII

1,2,3,4-Tetrahydro-5-phosphono-1-isoquinolinecarboxylic acid .

A solution of diethyl [1-(aminocarbonyl)-1,2,3,4-tetrahydro-5-isoquinolinyl]phosphonate (0.91 g, 2.9 mmol) in 6N HCl (50 ml) is heated at reflux for 18 hours. The reaction mixture is concentrated. The residue is twice dissolved in ethanol and concentrated to dryness. The resulting white solid is dried under vacuum. A white solid is obtained (0.78 g, 67%).

| Anal. calcd. for $C_{10}H_{12}NO_5P \cdot NH_4Cl \cdot HCl \cdot 0.8EtOH$: | | | | |
|---|---|---|---|---|
| | C, 36.28; | H, 5.72; | N, 7.30; | Cl, 18.47. |
| Found: | C, 36.36; | H, 5.83; | N, 7.66; | Cl, 17.31. |

Example VIII

1,2,3,4-Tetrahydro-7-[2-phosphonoethyl)-1-isoquinolinecarboxylic acid .

The corresponding compound of formula I is prepared from diethyl [2-[1-(aminocarbonyl)-1,2,3,4-tetrahydro-7-isoquinolinyl]ethyl]phosphonate in a process similar to Example I.

| Anal. calcd. for $C_{12}H_{16}NO_5P \cdot 0.15HCl \cdot 0.50H_2O$: | | | | |
|---|---|---|---|---|
| | C, 48.09; | H, 5.77; | N, 4.76; | Cl, 1.78. |
| Found: | C, 48.00; | H, 6.31; | N, 4.65; | Cl, 1.86. |

Example IX

1,2,3,4-Tetrahydro-7-phosphono-1-isoquinoline carboxylic acid .

A suspension of 2-ethyl-1-methyl 7-(diethoxyphosphinyl)-3,4-dihydro-1,2(1H)-isoquinolinedicarboxylate (2.63 g, 6.56 mmol) in aqueous 6N HCl is heated at reflux for 46 hours. The reaction mixture is decolorized with activated charcoal, filtered, and concentrated. The residue is placed under vacuum for 24 hours. The foam which remains is dissolved in water (15 ml) and freeze-dried. A white solid is obtained (0.93 g, 55%), mp 160-185° C (gas evolution).

| Anal. calcd. for $C_{10}H_{12}NO_5P \cdot HCl$: | | | | |
|---|---|---|---|---|
| | C, 40.90; | H, 4.46; | N, 4.77; | Cl, 12.07. |
| Found: | C, 40.55; | H, 4.53; | N, 4.43; | Cl, 11.60. |

Example X

1,2,3,4-Tetrahydro-6-(phosphonomethyl)-1-isoquinolinecarboxylic acid .

A solution of diethyl 6-[(diethoxyphosphinyl)methyl]-3,4-dihydro-1,2(1H)-isoquinolinedicarboxylate (0.16 g, 0.37 mmol) in 6N HCl (10 ml) is heated at reflux for 24 hours. The reaction mixture is concentrated and the residue is dissolved in water and freeze-dried. A solid is obtained (0.10 g, 99%).

| Anal. calcd. for $C_{11}H_{14}NO_5P \cdot 1.30HCl \cdot 1.30H_2O$: | | | | |
|---|---|---|---|---|
| | C, 38.63; | H, 5.28; | N, 4.10; | Cl, 13.47. |
| Found: | C, 38.73; | H, 4.55; | N, 4.15; | Cl, 13.35. |

Example XI

1,2,3,4-Tetrahydro-7-(phosphonomethyl)-1-isoquinolinecarboxylic acid .

The corresponding compound of formula I is sprepared from 2-ethyl-1-methyl 7-[(diethoxyphosphinyl)-methyl]-3,4-dihydro-1,2(1H)-isoquinolinedicarboxylate by a process analogous to Example I.

| Anal. calcd. for $C_{11}H_{14}NO_5P \cdot HCl$: | | | |
|---|---|---|---|
| | C, 42.94; | H, 4.91; | N, 4.55. |
| Found: | C, 42.86; | H, 5.16; | N, 4.05. |

Example XII

<u>1,2,3,4-Tetrahydro-5-(2-phosphonoethyl)benz[g]isoquinoline-3-carboxylic acid</u> .

The corresponding compound of formula I is prepared from triethyl 5-[2(diethoxyphosphinyl)ethyl]-1,4-dihydrobenz[g]isoquinoline-2,3,3-tricarboxylate in a process analogous to Example I.

Example XIII

<u>1,2,3,4-Tetrahydro-5-[2-(1H-tetrazol-5-yl)ethyl]-3-isoquinolinecarboxylic acid</u> .

The corresponding compound of formula I is prepared from triethyl 1,4-dihydro-5-[2-(1H-tetrazol-5-yl)-ethyl]-2,3,3-isoquinolinetricarboxylate in a process analogous to Example I.

## Claims

1. A compound of formula

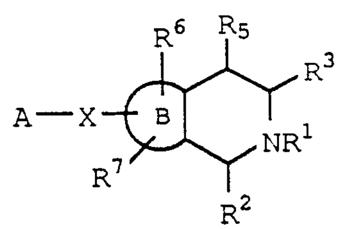

I

or a pharmaceutically acceptable acid addition or base salt thereof wherein
$R^1$ is hydrogen or a protecting group;
$R^2$ and $R^3$ are each independently hydrogen, alkyl, aryl, alkylaryl, or $COOR^4$ wherein $R^4$ is hydrogen, lower alkyl, lower alkenyl, aryl or arylalkyl, or a pharmacologically labile protecting group with the proviso that one of $R^2$ and $R^3$ must be $COOR^4$ and the other is hydrogen, alkyl, aryl, or alkylaryl;
$R^5$ is hydrogen or hydroxy when $R^3$ is $COOR^4$ and $R^5$ is hydrogen, alkyl, aryl or arylalkyl when $R^2$ is $COOR^4$;
$R^6$ and $R^7$ are each independently hydrogen, hydroxy, alkoxy, alkyl, aryl, arylalkyl, arylalkyloxy, halogen, trifluoromethyl or, when B is phenyl, are taken together with the ring carbons to which they are attached form a carbocyclic ring;
B is phenyl or a 5- or 6-membered heteroaryl ring;
X is $(CH_2)_n$-, -C=C-, $-OCH_2-$, $-OCH_2CH_2-$, $SCH_2-$, $SOCH_2$, $-SO_2CH_2-$, $-NHCH_2-$, $(CRR)_n$, $-(CH_2)_nCHR-$, $-CH_2CHRCH_2$, $-CHR(CH_2)_n-$, $CRR(CH_2)_n$, $-(CH_2)_nCRR-$, $-CH_2CRRCH_2-$, $-CHRCHRCH_2-$, $-CH_2CHRCHR$, $-CHRCH_2CHR-$, or n is an integer of from 0 to 2, R is hydrogen, hydroxy, or alkyl, and only one R on a carbon atom is hydroxy, and R and R may form a ring.
A is $-OPO_3R^8R^9$, $-PO_3R^8R^9$, $-PO_2R^8R^9$, $-BO_2R^8R^9$, $-CO_2R^8$ or $-SO_3R^8$ or tetrazole wherein $R^8$ and $R^9$ are

each independently hydrogen, alkyl, alkenyl, aryl, arylalkyl or a pharmaceutically acceptable labile group.

2. A compound according to Claim 1 wherein

$R^2$ and $R^3$ are each independently hydrogen, alkyl or $COOR^4$ wherein $R^4$ is hydrogen or lower alkyl or a pharmacologically labile protecting group with the proviso that one of $R^2$ and $R^3$ must be $COOR^4$ and the other of $R^2$ and $R^3$ is hydrogen or alkyl;

$R^6$ and $R^7$ are each independently hydrogen or when taken together with the ring carbons to which they are attached form $-CH=CH-CH=CH$;

X is $-(CH_2)_n$ wherein n is 0 to 3; and

A is $-PO_3R^8R^9$, $CO_2R^8$ or tetrazole wherein $R^8$ and $R^9$ are each independently hydrogen, lower alkyl, or a pharmaceutically acceptable labile group.

3. A compound according to Claim 1 and 2 wherein

$R^1$ is hydrogen;

$R^2$ and $R^3$ are each independently hydrogen or COOH;

$R^5$ is hydrogen; and

X is $(CH_2)_n$ wherein n is 0, 1 or 2.

4. A compound according to Claim 1 selected from the group consisting of:

1,2,3,4-tetrahydro-5-phosphono-3-isoquinolinecarboxylic acid,

1,2,3,4-tetrahydro-5-(phosphonomethyl)-3-isoquinolinecarboxylic acid,

1,2,3,4-tetrahydro-5-(2-phosphonoethyl)-3-isoquinolinecarboxylic acid,

1,2,3,4-tetrahydro-7-phosphono-1-isoquinolinecarboxylic acid,

1,2,3,4-tetrahydro-5-(2-phosphonoethyl)-benz[g]isoquinoline-3-carboxylic acid, and

1,2,3,4-tetrahydro-5-[2-(1H-tetrazol-5-yl)ethyl]-3-isoquinolinecarboxylic acid.

5. A pharmaceutical composition comprising a compound according to Claim 1 to 4 together with a pharmaceutically acceptable carrier.

6. Use of a compound of Claim 1 to 4 for the preparation of a pharmaceutical composition useful in treating cerebrovascular disorders or as an anesthetic in surgical operations where risk of cerebrovascular damage is finite.

7. A process for preparing a compound of Claim 1 to 4 wherein $R^3$ is COOH, X is $(CH_2)_n$, and n is 2 which comprises:

(1) reacting a compound of formula

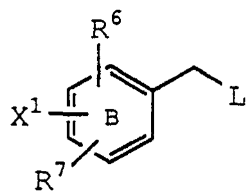

X

wherein L is a leaving group, $X^1$ is Br, I, $OSO_2CF_3$, and $R^6$ and $R^7$ are as defined above with diethylacetamidomalonate, dimethylacetamidomalonate, or diethylformamidomalonate, in the presence of sodium methoxide or sodium ethoxide in a solvent, over a period of 24 hours at room temperature to obtain a compound of

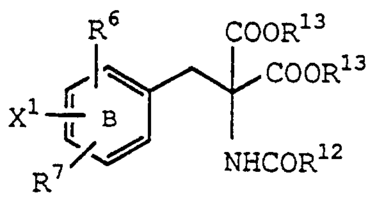

XI

wherein $R^{12}$ is lower alkyl, aryl, arylalkyl, and $R^{13}$ is lower alkyl or arylalkyl;

(2) treating a compound of the formula XI of Step (1) under aqueous acid conditions over a period of 24 to 48 hours at temperature from 80 °C to reflux to obtain a compound

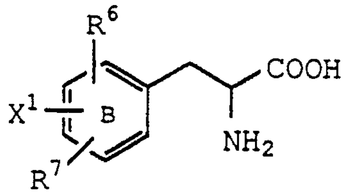 ; XII

(3) treating a compound of formula XII from Step (2) with ethylchloroformate, methylchloroformate, benzylchloroformate at room temperature in a solvent over a period of 1 to 24 hours to obtain a compound of the formula

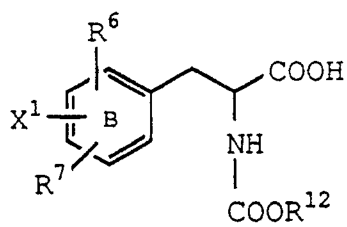 ; XIII

(4) treating a compound of formula XIII from Step (3) with an esterifying reagent at room temperature in a solvent to obtain a compound of

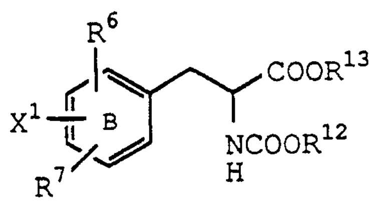 XIV

wherein $R^{13}$ is lower alkyl, aryl, or arylalkyl;

(5) treating a compound of formula XIV from Step (4) with an aldehyde in a solvent to obtain a compound of

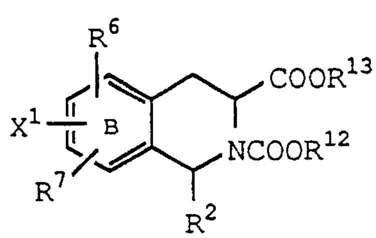 ; XV

(6) treating a compound of formula XV from Step (5) above with a compound of the formula (XVI)

$H_2C=CHD$ XVI

wherein D is CN, $CO_2R^{12}$, or $PO(OR^1_a)(OR^2_b)$ and $R^1_a$ and $R^2_b$ are lower alkyl in the presence of Palladium II reagent and a trialkylamine in a solvent to obtain a compound of formula

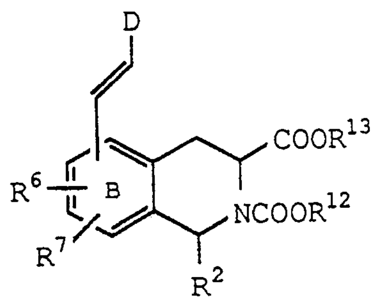

; XVII

(7) hydrogenating a compound of formula XVII from step (6) in a solvent to give a compound of the XVIII.

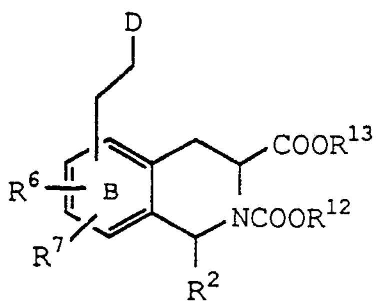

XVIII

(8) deprotecting a compound of formula XVIII from Step (7) over a period of 10-72 hours to obtain a compound of formula I.

8. A process for the preparation of a compound according Claim 1 to 4 wherein $R^2$ is hydrogen, $R^3$ is COOH, X is $(CH^2)_n$ and n is 0 comprising:

(1) reacting a compound of formula XV with $HPO(OR^1{}_a)(OR^2{}_b)$ in the presence of a palladium (O) reagent and a trialkylamine in a solvent to give the compound

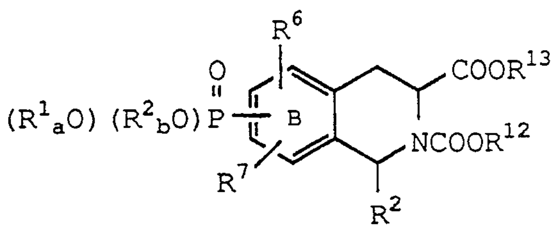

XX

(2) deprotecting a compound of the formula XX from Step (1) to obtain formula I.

9. A process for the preparation of a compound according to Claim 1 to 4 wherein $R^3$ is COOH and X is $(CH_2)_n$ wherein n is 1 or 2 comprising

(1) reacting a compound of the formula

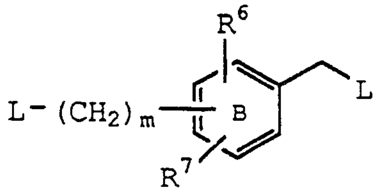

XXIII

wherein L is a leaving group, $R^6$ and $R^7$ are as defined above, and m is 1 with a compound of formula $R^{10}D$

wherein $R^{10}$ is sodium or potassium and D is $PO(OR^1{}_a)(OR^2{}_b)$ or CN in a solvent to give a compound of

formula XXIV

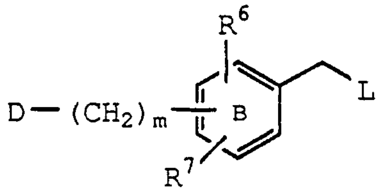

; XXIV

and;

(2) reacting sodium ethoxide in ethanol with N-carboethoxydiethyl aminomalonate and a compound of formula XXIV to obtain a compound of formula XXV

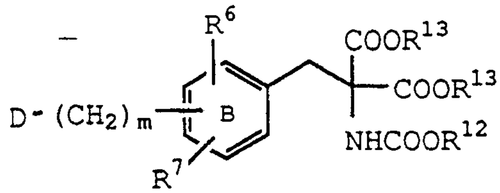

XXV

10. A process for the preparation of a compound according to Claim 1 to 4 comprising:

(1) treating a compound of formula XXIII wherein m is 2 with N-carboethoxydiethylaminomalonate in the presence of sodium ethoxide in ethanol to afford the compound of formula XXVI

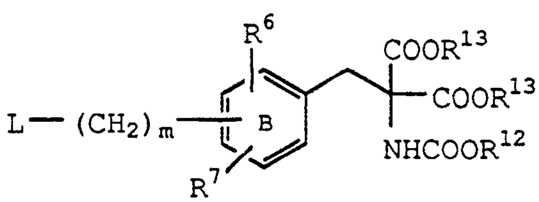

XXVI

(2) treating a compound of formula XXVI from Step (1) wherein m is 1 or 2 with a compound of the formula $R^{10}D$ wherein $R^{10}$ and D are as defined above in a solvent to afford a compound of formula XXV;

(3) treating a compound of formula XXV with an aldehyde in a solvent at room temperature for a period of 1-48 hours to obtain a compound of formula XXVIII

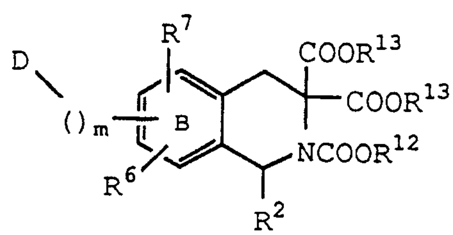

XXVIII

(4) deprotecting a compound of formula XXVIII from step (3) over a period of 10-72 hours to obtain the compound of formula I.

11. A process for the preparation of a compound according to Claim 1 to wherein $R^3$ is COOH, X is $(CH_2)_n$, and n is 2, comprising

(1) treating a compound of formula XXVI

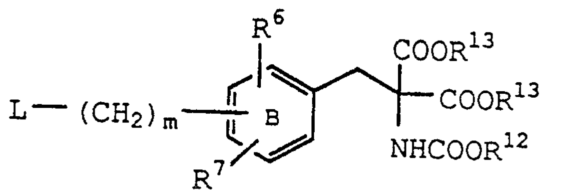

XXVI

with

$H_2C=CHD$ XVI

in the presence of a palladium catalyst and a trialkylamine in a solvent to obtain a compound of formula XXVII

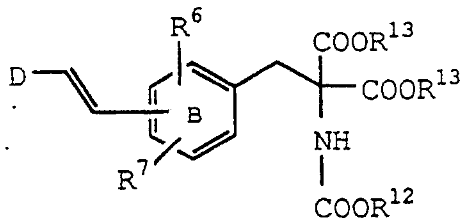

XXVII

and by hydrogenating the product to produce a compound of formula XXV

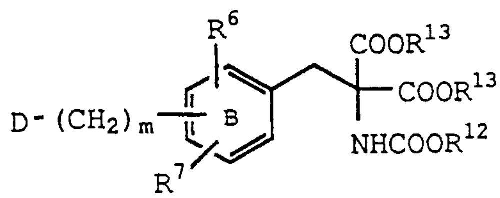

XXV

12. A process for the preparation of a compound XXXVIII

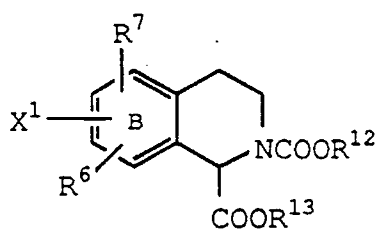

XXXVIII

wherein $X^1$ is chloro, bromo or iodo, $R^6$, $R^7$, $R^{12}$, and $R^{13}$ are as defined above, which comprises reacting a compound of formula XXXVII

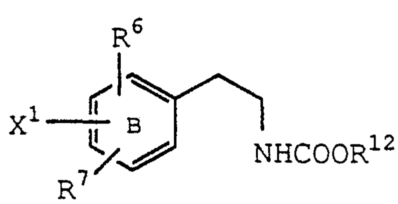

XXXVII

with glyoxylic acid or an ester thereof in a solvent to obtain a desired compound.

13. A compound selected from the group consisting of:

Dimethyl 5-bromo-3,4-dihydro-2,3-(1H)-isoquinolinedicarboxylate,

Dimethyl 7-bromo-3,4-dihydro-2,3-(1H)-isoquinolinedicarboxylate,
Dimethyl 5-(diethoxyphosphinyl)-3,4-dihydro-2,3(1H)-isoquinolinedicarboxylate,
Dimethyl 7-(diethoxyphosphinyl)-3,4-dihydro-2,3(1H)-isoquinolinedicarboxylate,
Dimethyl 5-[2-(diethoxyphosphinyl)ethenyl]-3,4-dihydro-2,3(1H)-isoquinolinedicarboxylate,
Dimethyl 7-[2-(diethoxyphosphinyl)ethenyl]-3,4-dihydro-2,3(1H)-isoquinolinedicarboxylate,
Dimethyl 5-[2-(diethoxyphosphinyl)ethyl]-3,4-dihydro-2,3(1H)-isoquinolinedicarboxylate,
Dimethyl 7-[2-(diethoxyphosphinyl)ethyl]-3,4-dihydro-2,3(1H)-isoquinolinedicarboxylate,
Triethyl 1,4-dihydro-5-[(diethoxyphosphinyl)methyl]-2,3,3-isoquinolinetricarboxylate,
Triethyl 1,4-dihydro-7-[(diethoxyphosphinyl)methyl]-2,3,3-isoquinolinetricarboxylate,
7-Bromo-3,4-dihydro-1,2(1H)-isoquinolinedicarboxylic acid, 2-ethyl ester,
2-Ethyl-1-methyl-7-bromo-3,4-dihydro-1,2(1H)isoquinolinedicarboxylate,
2-Ethyl-1-methyl-7-(diethoxyphosphinyl)-3,4-dihydro-1,2(1H)-isoquinolinedicarboxylate,
Diethyl [[3-(2-aminoethyl)phenyl]methyl]phosphonate,
Diethyl [[4-(2-aminoethyl)phenyl]methyl]phosphonate,
Ethyl [2-[3-(diethoxyphosphinyl)phenyl]ethyl]carbamate,
Ethyl [2-[4-(diethoxyphosphinyl)phenyl]ethyl]carbamate,
6-[(Diethoxyphosphinyl)methyl]-3,4-dihydro-1,2(1H)-isoquinolinedicarboxylic acid, 2-ethyl ester,
7-[(Diethoxyphosphinyl)methyl]-3,4-dihydro-1,2(1H)-isoquinolinedicarboxylic acid, 2-ethyl ester,
Diethyl 6-[(diethoxyphosphinyl)methyl]-3,4-dihydro-1,2(1H)-isoquinolinedicarboxylate,
2-Ethyl-1-methyl-7-[(diethoxyphosphinyl)methyl]-3,4-dihydro-1,2(1H)-isoquinolinedicarboxylate,
Diethyl 5-isoquinolinylphosphonate, N-oxide,
Diethyl 7-isoquinolinylphosphonate, N-oxide,
Diethyl [2-(7-isoquinolinyl)ethyl]phosphonate, N-oxide,
Diethyl (1-cyano-5-isoquinolinyl)phosphonate,
Diethyl (1-cyano-7-isoquinolinyl)phosphonate,
Diethyl [2(1-cyano-7-isoquinolinyl)ethyl]phosphonate,
Diethyl [1-(aminocarbonyl)-5-isoquinolinyl]phosphonate,
Diethyl [1-(aminocarbonyl)-7-isoquinolinyl]phosphonate,
Diethyl [2-[1-(aminocarbonyl)-7-isoquinolinyl]ethyl]phosphonate,
Diethyl [1-(aminocarbonyl)-1,2,3,4-tetrahydro-5-isoquinolinyl]phosphonate,
Diethyl [1-(aminocarbonyl)-1,2,3,4-tetrahydro-7-isoquinolinyl]phosphonate,
Diethyl [2-[1-(aminocarbonyl)-1,2,3,4-tetrahydro-7-isoquinolinyl]ethyl]phosphonate,
Diethyl [[1-[2(diethoxyphosphinyl)ethenyl]-Z-naphthalenyl]methyl] [(ethoxycarbonyl)amino]propanedioate,
Diethyl [[1-[2-(diethoxyphosphinyl)ethyl]-Z-naphthalenyl]methyl][(ethoxycarbonyl)amino]propanedioate,
Triethyl 5[2-(diethoxyphosphinyl)ethyl]-1,4-dihydrobenz[g]isoquinoline-2,3,3-tricarboxylate,
Diethyl [[2-(2-cyanoethyl)phenyl]methyl][(ethoxycarbonyl)amino]propanedioate,
Triethyl 5-(2-cyanoethyl)-1,4dihydro-2,3,3-isoquinolinetricarboxylate, and
Triethyl 1,4-dihydro-5-[2-(1H-tetrazol-5-yl)ethyl]2,3,3-isoquinolinetricarboxylate.